# FASCICULE DE BREVET EUROPEEN

(11) **EP 3 621 436 B1**
(45) Date de publication et mention de la délivrance du brevet: **31.01.2024**
(21) Numéro de dépôt: 18737330.3
(22) Date de dépôt: 14.05.2018
(51) Int. Cl.: A01N 25/04, A61P 17/10, A61K 8/23, A61K 8/19, A61Q 19/00, A61K 9/06, A61K 33/38, A61K 33/34, A61K 33/24, A61K 33/04, A01P 3/00, A01P 1/00, A01N 59/20, A01N 59/16, A61K 33/242

(54) **NOUVEAUX BACTÉRICIDES ET ANTIFONGIQUES**
NEUARTIGE BAKTERIZIDE UND ANTIMYKOTIKA
NOVEL BACTERICIDES AND ANTIFUNGAL AGENTS

(30) Priorité: 12.05.2017 FR 1754219
(43) Date de publication de la demande: 18.03.2020
(73) Titulaire: Walter, Jean-Jacques, 75006 Paris (FR)
(72) Inventeur: Walter, Jean-Jacques, 75006 Paris (FR)
(74) Mandataire: Grosset-Fournier, Chantal Catherine
(86) Numéro de dépôt international: PCT/FR2018/000117
(87) Numéro de publication internationale: WO 2018/206867

(56) Documents cités:
- WO-A1-2005/077329
- US-A1- 2006 246 149
- US-A1- 2008 233 005
- US-A1- 2010 180 413
- US-A1- 2013 142 886
- US-A1- 2016 038 413
- DATABASE GNPD [Online] MINTEL; 1 octobre 2016 (2016-10-01), "Miracellar Cleansing Water", XP002778168, Database accession no. 4323129
- DATABASE GNPD [Online] MINTEL; 1 septembre 2015 (2015-09-01), "Gentle Exfoliating Cleansing Cream", XP002778169, Database accession no. 3376195
- DATABASE GNPD [Online] MINTEL; 1 août 2015 (2015-08-01), "Repairing Cream", XP002778170, Database accession no. 3392947
- Juneyoung Lee ET AL: "The Silver Nanoparticle (Nano-Ag): a New Model for Antifungal Agents" In: "Silver Nanoparticles", 1 mars 2010 (2010-03-01), InTech, XP055450559, ISBN: 978-953-30-7028-5 DOI: 10.5772/8510, tableau 1 sections 2.2, 2.4, 2.5, 3.1, 4
- M F ZAWRAH ET AL: "Antimicrobial Activities of Gold Nanoparticles against Major Foodborne Pathogens", LIFE SCIENCE JOURNAL LIFE SCIENCE JOURNAL, vol. 8, 1 janvier 2011 (2011-01-01), XP055450763,
- EBENEZER ADDAE ET AL: "Investigation of antimicrobial activity of photothermal therapeutic gold/copper sulfide core/shell nanoparticles to bacterial spores and cells", JOURNAL OF BIOLOGICAL ENGINEERING, BIOMED CENTRAL LTD, LO, vol. 8, no. 1, 2 juin 2014 (2014-06-02), page 11, XP021189648, ISSN: 1754-1611, DOI: 10.1186/1754-1611-8-11

## Description

La présente invention concerne la production de nouveaux bactéricides et fongicides.

Il existe un nombre croissant de bactéries devenues résistantes aux antibiotiques connus, et la recherche de nouveaux antibiotiques piétine.

Il existe d'autre part des formations bactériennes nommées biofilms, qui sont résistantes à tous les antibiotiques connus.

Les produits cosmétiques et certains médicaments sont formés d'une émulsion pâteuse d'eau et d'huile. Cette émulsion risque d'être colonisée par des bactéries et des moisissures. On les protège en adjoignant des parabènes en tant que conservateur, à raison de 0,4% à 0,8%. Les parabènes ont des inconvénients qui conduisent à chercher à la remplacer.

Par ailleurs, dans leur utilisation comme anti-acné, les médicaments actuels (Epiduo, Aczone, Finacea, Acanya, and Ziana) présentent de sérieux inconvénients : ils provoquent une sécheresse de la peau, des démangeaisons, des desquamations et un léger oedème, ce qui conduit souvent à l'abandon du traitement. De plus ils sont tératogènes sur les foetus des femmes enceintes qui utilisent l'un de ces anti-acnés actuels.

L'un des buts de l'invention est donc fournir de nouveaux bactéricides.

L'un des buts de l'invention est donc fournir de nouveaux antifongiques
Un autre but de l'invention est d'utiliser un métal de transition tel que revendiqué dans le traitement de l'acné

La présente invention concerne l'utilisation comme bactéricide ou comme anti fongique d'un métal de transition, ou l'utilisation d'un métal de transition tel que revendiqué dans le traitement de maladies bactériennes ou fongiques, notamment l'acné.

Par « *utilisation comme bactéricide ou utilisation dans le traitement de maladies bactériennes* » il est entendu, au sens de la présente invention, une utilisation dans le but de tuer au moins une bactérie. Les bactéries concernées sont des bactéries Gram positif ou Gram négatif, en particulier, les bactéries concernées sont choisies parmi *Staphylococcus Aureus, Staphylococcus aureus résistant à la méticilline (MRSA), Staphylococcus aureus sensible à la méticilline (MSSA), Propionibacterium Acnes, Escherichia Coli Pseudomonas Aeruginas,*

Par « *utilisation comme antifongique ou utilisation dans le traitement de maladies fongiques»* il est entendu, au sens de la présente invention, une utilisation dans le but de tuer au moins une levure par exemple Candida Albicans

Au sens de la présente invention, il est entendu d'un « *traitement de l'acné »* une action bactéricide sur une bactérie responsable de l'acné. En particulier, le traitement de l'acné s'entend d'une action bactéricide sur *Propionibacterium Acnes,* ou d'une action à la fois sur *Propionibacterium Acnes* et sur *Staphylococcus Aureus.*

Au sens de la présente invention, un bactéricide ou antifongique s'entend d'une substance dont la Concentration Minimale Inhibitrice est inférieure ou égale à 0,2 g/L, comprise entre 0,1 et 0,2 g/L ou inférieure ou égale à 0,1 g/L.

Un premier objet de l'invention est un métal de transition sous forme insoluble, notamment sous forme d'un métal de transition au degré d'oxydation 0 ou sous forme de sulfure ou d'un oxyde du métal de transition,
ledit métal de transition étant choisi parmi l'or, l'argent et le cuivre,
ledit métal de transition sous forme insoluble étant mis en solution aqueuse par la formation de nanoparticules par ajout d'un surfactant,
ledit métal de transition étant contenu dans une structure constituée d'un coeur contenant ledit métal de transition, entouré d'une coquille de surfactant,
pour son utilisation dans le traitement de maladies bactériennes ou fongiques.

Au sens de l'invention, il est entendu par « *métal de transition* » un noyau choisi parmi la colonne IB.

Au sens de la présente invention, il est entendu par « *utilisation d'un métal de transition »* l'utilisation d'au moins un métal de transition. La présente description concerne donc également l'utilisation d'un mélange de métaux de transition, en particulier un mélange de deux ou trois métaux de transition.

Par « *insoluble* » est entendue, au sens de l'invention, une poudre qui ne se dissout pas dans l'eau, c'est-à-dire dont la concentration maximale en solution aqueuse est inférieure à 0,5 mg.L⁻¹ à température ambiante, c'est-à-dire à une température d'environ 25 C, et qui est mise en solution aqueuse par l'ajout d'un additif pour former des nanoparticules ou des complexes.

Dans le cas des nanoparticules, l'additif est un surfactant. Des exemples de surfactants incluent les RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, notamment les R1triméthylammoniumX. Le surfactant peut aussi être un bromure d'ammonium triméthylR1 dans lequel R1 représente un alkyle de 6 à 20 atomes de carbone et notamment de 8, 9, 10, 11, 12, 13, 14, 15 , 16, 17, ou 18 atomes de carbone, ou également un polyéthylène glycol, ou un polyéthylène glycol-dithiol.

Au sens de la présente invention, on entend par « acide minéral », un acide dérivant d'un corps minéral ou inorganique, tel que les acides chlorhydrique, sulfurique, ou nitrique.

Au sens de la présente invention, on entend par « acide organique », un composé organique présentant des propriétés acides, tel que les acides carboxyliques de formule R2COOH, R2 étant un hydrogène ou un composé organique.

Au sens de la présente invention, l'expression «alkyle en C1 à C20» désigne une chaîne carbonée acyclique, saturée, linéaire ou ramifiée, comprenant 1 à 20 atomes de carbone. Des exemples d'alkyles en C1 à C20 incluent les groupes méthyle, éthyle, propyle, butyle, pentyle, hexyle ou heptyle. La définition de propyle, butyle, pentyle, hexyle ou heptyle inclut tous les isomères possibles. Par exemple, le terme butyle comprend n-butyle, iso-butyle, sec-butyle et tert-butyle. L'alkyle peut être substitué à différentes positions par un ou plusieurs groupements fonctionnels tels que halogène, alcoxyle, amino, nitro, cyano, trifluorométhyle ou ester carboxylique.

Dans le cas des complexes, l'additif est un agent de solubilisation choisi parmi l'ammoniaque, l'acide citrique, le bichromate de potassium ou un mélange de ceux-ci. Lorsque l'agent de solubilisation est un mélange d'ammoniaque et d'acide citrique, du citrate d'ammonium est formé ; l'agent de solubilisation ainsi formé est alors soit du citrate d'ammonium avec de l'acide citrique si l'acide citrique est présent en excès molaire par rapport à l'ammoniaque, soit du citrate d'ammonium avec de l'ammoniaque si l'ammoniaque est introduit en excès molaire par rapport à l'acide citrique.

Sous la forme insoluble du substitut de parabène de l'invention sont donc regroupés les nanoparticules et les complexes de métaux de transition tels que définis ci-dessus.

Un sel « *soluble* », au sens de l'Invention, est à l'inverse, une poudre qui se solubilise au contact de l'eau, sans ajout d'un additif, c'est-à-dire dont la concentration maximale en solution aqueuse est supérieure à 0,5 mg.L⁻¹. Sous la forme soluble du substitut de parabène de la description est comprise les sels et oxydes de métaux de transition tels que définis ci-dessus.

Au sens de la présente invention le terme « *mis en solution aqueuse* » correspond à la formation d'une solution, d'une émulsion ou d'une suspension aqueuse dont la concentration en métal de transition tel que décrit ci-dessus est supérieure à 0,5 mg.L⁻¹.

Au sens de la présente description, il est entendu par « *complexe* » un cation métallique, formé à partir d'un métal de transition tel que défini précédemment, lié à plusieurs atomes, ions ou molécules appelés ligands. Des exemples de ligands incluent l'eau et les additifs définis ci-dessus.

Selon un mode de réalisation avantageux, l'invention concerne un métal de transition tel que décrit ci-dessus, sous forme insoluble, ledit métal de transition sous forme insoluble étant mis en solution aqueuse par la formation de nanoparticules, pour son utilisation dans le traitement de l'acné.

Selon un mode de réalisation particulier, l'invention concerne un métal de transition tel que décrit ci-dessus, contenu dans une structure constituée d'un coeur contenant ledit métal de transition, entouré d'une coquille de surfactant, ledit surfactant étant notamment choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, un polyéthylène glycol, ou un polyéthylène glycol-dithiol, pour son utilisation dans le traitement de maladies bactériennes ou fongiques, notamment l'acné.

Au sens de la présente invention, il est entendu par «surfactant » une molécule qui présente une extrémité hydrophile et une autre extrémité hydrophobe.

Comme défini précédemment, le surfactant peut être une bromure d' ammonium triméthyl R1 dans lequel R1 représente un alkyle de 6 à 20 atomes de carbone et notamment de 8, 9, 10, 11, 12, 13, 14, 15 ,16, 17, ou 18 atomes de carbone. Des exemples de surfactants incluent le CTAB (bromure de cétyltriméthylammonium ; Br[N(CH₃)₃(C₁₆H₃₃)]), le OTAB (le bromure d'octyltriméthylammonium ; Br[N(CH₃)₃(C₈H₁₇)]), RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, notamment les R 1 triméthylammoniumX, ou le PEG-dithiol (Polyéthylène glycol comprenant des fonctions thiols terminales) et le PEG-600 (Polyéthylène glycol dont la masse molaire moyenne est de 600 g/mol). De préférence, le surfactant utilisé pour stabiliser les nanoparticules est le CTAB, ou CTAX, X étant un acide, de préférence fort, notamment l'acide nitrique ou sulfurique.

Les nanoparticules ayant une structure constituée d'un coeur contenant le métal de transition, entouré d'une coquille de surfactant, mesurent environ 2 à 4 nanomètres de diamètre. Le diamètre du coeur représente environ 60% du diamètre total, et l'épaisseur de la coquille environ 40% du diamètre total. La masse du coeur représente environ 57% de la masse totale de la nanoparticule et la coquille environ 43% de la masse totale.

Au sens de la présente invention, le métal de transition de la colonne IB de Mendeleïev est au degré d'oxydation 0, +I, +II ou +III. En particulier, l'Invention concerne l'utilisation d'un métal de transition choisi parmi Cu(0), Cu(II), Ag(0), Ag(I), Au(0) ou Au(III).

Selon un mode de réalisation particulier, l'invention concerne un métal de transition tel que décrit ci-dessus, choisi parmi le cuivre, l'argent et l'or, étant au degré d'oxydation 0 et sous forme de nanoparticules, pour son utilisation dans le traitement de maladies bactériennes ou fongiques, notamment l'acné.

Selon un mode de réalisation avantageux, l'invention concerne un métal de transition tel que décrit ci-dessus, dans une composition contenant de l'eau, notamment comprenant de l'ordre de 1 à 200 mg, en particulier 1 à 5, 5 à 10, 10 à 50, 50 à 75, 75 à 100, 100 à 125, 125 à 150, 150 à 175, 175 à 200 mg, de métal de transition par litre de composition, pour son utilisation dans le traitement de maladies bactériennes ou fongiques, notamment l'acné.

Selon un mode de réalisation particulier, l'invention concerne un métal de transition dans une composition telle que définie ci-dessus, ladite composition contenant de l'eau étant une solution aqueuse, pour son utilisation dans le traitement de maladies bactériennes ou fongiques, notamment l'acné.

Selon un mode de réalisation particulier, l'invention concerne un métal de transition dans une composition telle que définie ci-dessus, ladite composition contenant de l'eau étant une émulsion ou une suspension, pour son utilisation dans le traitement de maladies bactériennes ou fongiques, notamment l'acné.

Selon un mode de réalisation particulier, l'invention concerne un métal de transition dans une composition telle que définie ci-dessus, ladite composition contenant de l'eau étant une émulsion huile-dans-l'eau ou eau-dans-l'huile, notamment comprenant du cuivre, de l'argent ou de l'or sous la forme de nanoparticules, pour son utilisation dans le traitement de maladies bactériennes ou fongiques, notamment l'acné.

Un autre objet de l'invention est un métal de transition sous forme insoluble, notamment sous forme d'un métal de transition au degré d'oxydation 0 ou sous forme de sulfure ou d'un oxyde du métal de transition,
ledit métal de transition étant choisi parmi l'or, l'argent et le cuivre,
ledit métal de transition sous forme insoluble étant mis en solution aqueuse par la formation de nanoparticules par ajout d'un surfactant,
ledit métal de transition étant contenu dans une structure constituée d'un coeur contenant ledit métal de transition, entouré d'une coquille de surfactant,
pour son utilisation comme antibactérien ou antifongique.

Selon un mode de réalisation particulier, l'invention concerne un métal de transition tel que décrit ci-dessus, sous forme insoluble, ledit métal de transition sous forme insoluble étant mis en solution aqueuse par la formation de nanoparticules, pour son utilisation comme antibactérien ou antifongique, notamment comme anti-acnéique.

Selon un mode de réalisation particulier, l'invention concerne un métal de transition tel que décrit ci-dessus, contenu dans une structure constituée d'un coeur contenant ledit métal de transition, entouré d'une coquille de surfactant, ledit surfactant étant notamment choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, un polyéthylène glycol, ou un polyéthylène glycol-dithiol, pour son utilisation comme antibactérien ou antifongique, notamment comme anti-acnéique.

Selon un mode de réalisation particulier, l'invention concerne un métal de transition tel que décrit ci-dessus, dans une composition contenant de l'eau, notamment comprenant de l'ordre de 1 à 200 mg, en particulier 1 à 5, 5 à 10, 10 à 50, 50 à 75, 75 à 100, 100 à 125, 125 à 150, 150 à 175, 175 à 200 mg, de métal de transition par litre de composition, pour son utilisation comme antibactérien ou antifongique, notamment comme anti-acnéique.

Selon un mode de réalisation particulier, l'invention concerne un métal de transition dans une composition telle que définie ci-dessus, ladite composition contenant de l'eau étant une solution aqueuse, pour son utilisation comme antibactérien ou antifongique, notamment comme anti-acnéique.

Selon un mode de réalisation particulier, l'invention concerne un métal de transition dans une composition telle que définie ci-dessus, ladite composition contenant de l'eau étant une émulsion ou une suspension, pour son utilisation comme antibactérien ou antifongique, notamment comme anti-acnéique.

Selon un mode de réalisation particulier, l'invention concerne un métal de transition dans une composition telle que définie ci-dessus, ladite composition contenant de l'eau étant une émulsion huile-dans-l'eau ou eau-dans-l'huile, et notamment comprenant du cuivre, de l'argent ou de l'or sous la forme de nanoparticules, pour son utilisation comme antibactérien ou antifongique, notamment comme anti-acnéique.

Un autre objet de l'invention est une composition pharmaceutique ou vétérinaire contenant un métal de transition tel que décrit ci-dessus.

Selon un mode de réalisation particulier, l'invention concerne une composition pharmaceutique ou vétérinaire telle que décrite ci-dessus, dans laquelle le métal de transition est à une dose de 1 mg à 1 g, en particulier 1 à 10 mg, 10 à 50 mg, 50 à 100 mg, 100 à 500 mg, 500 mg à 1 g, de métal de transition par litre de composition.

Selon un mode de réalisation particulier, l'invention concerne une composition pharmaceutique ou vétérinaire telle que décrite ci-dessus, comprenant comme excipients au moins une huile végétale, notamment de l'huile d'argan, de pépins de raisin, d'avocat, de chanvre ou de noyaux d'abricots.

Selon un mode de réalisation particulier, l'invention concerne une composition pharmaceutique ou vétérinaire telle que décrite ci-dessus, dont la forme d'administration est topique.

Selon un mode de réalisation particulier, l'invention concerne une composition pharmaceutique ou vétérinaire telle que décrite ci-dessus, comprenant comme excipients au moins une huile végétale, dont la forme d'administration est topique.

Au sens de l'invention, il est entendu par « administration topique », une administration sur un point externe du corps, ou sur une surface du corps telle que la peau ou les muqueuses, ayant une action directe au point d'administration.

Un autre objet de l'invention est l'utilisation d'un métal de transition tel que décrit ci-dessus pour un usage cosmétique.

Un autre objet de l'invention est une composition cosmétique contenant un métal de transition tel que décrit ci-dessus.

Selon un mode de réalisation particulier, l'invention concerne une composition cosmétique telle que décrite ci-dessus, comprenant de l'ordre de 1 à 200 mg, en particulier 1 à 5, 5 à 10, 10 à 50, 50 à 75, 75 à 100, 100 à 125, 125 à 150, 150 à 175, 175 à 200 mg, de métal de transition par litre de composition.

Aussi décrite est une méthode de traitement de maladies bactériennes ou fongiques, notamment l'acné, comprenant l'administration par voie topique d'un métal de transition tel que décrit ci-dessus sous forme insoluble, ledit métal de transition sous forme insoluble étant mis en solution aqueuse par la formation de nanoparticules.

Selon un mode de réalisation particulier, l'invention concerne une méthode de traitement de maladies bactériennes ou fongiques, notamment l'acné, comprenant l'administration par voie topique d'un métal de transition tel que décrit ci-dessus contenu dans une structure constituée d'un coeur contenant ledit métal de transition, entouré d'une coquille de surfactant, ledit surfactant étant notamment choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, un polyéthylène glycol, ou un polyéthylène glycol-dithiol.

Aussi décrite est une méthode de traitement de maladies bactériennes ou fongiques, comprenant l'administration par voie topique sur l'Homme ou l'animal, en particulier le cheval, d'un métal de transition sous forme insoluble, notamment sous forme d'un métal de transition au degré d'oxydation 0 ou sous forme de sulfure ou d'un oxyde du métal de transition.

Aussi décrite est une méthode de traitement de maladies bactériennes ou fongiques, comprenant l'administration par voie topique sur l'Homme ou l'animal, en particulier le cheval, d'un métal de transition tel que décrit ci-dessus sous forme insoluble, ledit métal de transition sous forme insoluble étant mis en solution aqueuse par la formation de nanoparticules.

Aussi décrite est une méthode de traitement de maladies bactériennes ou fongiques, comprenant l'administration par voie topique sur l'Homme ou l'animal, en particulier le cheval, d'un métal de transition tel que décrit ci-dessus contenu dans une structure constituée d'un coeur contenant ledit métal de transition, entouré d'une coquille de surfactant, ledit surfactant étant notamment choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, un polyéthylène glycol, ou un polyéthylène glycol-dithiol.

Aussi décrite est une méthode de traitement de maladies bactériennes ou fongiques, comprenant l'administration par voie topique sur l'Homme ou l'animal, en particulier le cheval, d'un métal de transition tel que décrit ci-dessus, choisi dans la colonne IB de Mendeleïev, choisi parmi le cuivre, l'argent et l'or.

Un autre but de l'invention est de remplacer le parabène présent comme conservateur dans les compositions alimentaires, cosmétiques et/ou pharmaceutique.

Aussi décrit est d'utiliser un métal de transition pour remplacer le parabène présent comme conservateur dans les compositions alimentaires, cosmétiques et/ou pharmaceutique.

Aussi décrite est une composition dépourvue de parabène et comprenant des sulfures de métaux de transition utilisés comme conservateur.

Aussi décrite est l'utilisation comme substitut de parabène d'un métal de transition.

Il est entendu, par « *parabène* », un parahydroxybenzoate d'alkyle c'est-à-dire un ester, possédant des propriétés bactéricides et fongicides à large spectre. Les propriétés antibactériennes et antifongiques des parabènes font qu'ils sont généralement utilisés comme conservateur dans les cosmétiques, les médicaments et les aliments.

Par « *utilisation comme substitut de parabène* » il est entendu, une utilisation pour remplacer les parabènes utilisés comme conservateur en raison de leurs propriétés antibactériennes et/ou antifongiques.

Aussi décrite est l'utilisation comme substitut de parabène, d'un métal de transition sous forme insoluble, notamment sous forme d'un métal de transition au degré d'oxydation 0 ou sous forme de sulfure ou d'un oxyde du métal de transition.

Dans le cas des nanoparticules, l'additif est un surfactant. Des exemples de surfactants incluent les RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, notamment les R1triméthylammoniumX. Le surfactant peut aussi être un bromure d'ammonium triméthylR dans lequel R1 représente un alkyle de 6 à 20 atomes de carbone et notamment de 8, 9, 10, 11, 12, 13, 14, 15 ,16, 17, ou 18 atomes de carbone. Des exemples de surfactants incluent le CTAB (bromure de cétyltriméthylammonium ; Br[N(CH₃)₃(C₁₆H₃₃)]), le OTAB (le bromure d'octyltriméthylammonium ; Br[N(CH₃)₃(C₈H₁₇)]), PEG-dithiol (Polyéthylène glycol comprenant des fonctions thiols terminales) et le PEG-600 (Polyéthylène glycol dont la masse molaire moyenne est de 600 g/mol).

Aussi décrite est l'utilisation comme substitut de parabène, d'un métal de transition sous forme insoluble, notamment sous forme d'un métal de transition au degré d'oxydation 0, sous réserve que lorsque le métal est de l'argent, celui-ci ne soit pas déposé à la surface d'un polymère, ou sous forme de sulfure ou d'un oxyde du métal de transition.

Aussi décrite est l'utilisation comme substitut de parabène, d'un métal de transition sous forme insoluble, notamment sous forme d'un métal de transition au degré d'oxydation 0, notamment choisi dans la colonne IB de Mendeleïev choisi parmi le cuivre, l'argent et l'or, sous réserve que lorsque le métal est de l'argent, celui-ci ne soit pas déposé à la surface d'un polymère,

Aussi décrite est l'utilisation comme substitut de parabène, d'un métal de transition sous forme insoluble, notamment sous forme de sulfure ou d'un oxyde du métal de transition, notamment choisi dans la colonne IB de Mendeleïev, choisi parmi le cuivre, l'argent et l'or.

Aussi décrite est l'utilisation comme substitut de parabène, d'un métal de transition sous forme soluble notamment des sels de sulfates, nitrates ou chlorures du métal de transition, notamment choisi dans la colonne IB de Mendeleïev, en particulier choisi parmi le cuivre, l'argent et l'or.

Selon un mode de réalisation, le diamètre des nanoparticules de l'invention est de 1 à 10 nm, notamment de 1 à 5 nm, en particulier de 2 nm à 4 nm.

Aussi décrie est l'utilisation comme substitut de parabène, d'un métal de transition sous forme insoluble, ledit métal de transition sous forme insoluble étant mis en solution aqueuse par la formation de nanoparticules.

Aussi décrite est l'utilisation comme substitut de parabène, d'un métal de transition, ledit métal de transition étant contenu dans une structure constituée d'un coeur contenant ledit métal de transition, entouré d'une coquille de surfactant, ledit surfactant étant notamment choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, un polyéthylène glycol, ou un polyéthylène glycol-dithiol.

Aussi décrite est l'utilisation comme substitut de parabène d'un métal de transition choisi dans la colonne IB de Mendeleïev, choisi parmi le cuivre, l'argent et l'or.

Aussi décrite est l'utilisation comme substitut de parabène d'un métal de transition choisi parmi le cuivre, l'argent et l'or, et étant sous forme de sulfure.

Aussi décrite est l'utilisation comme substitut de parabène d'un métal de transition choisi parmi le cuivre, l'argent et l'or, étant sous forme de sulfure et sous forme d'un complexe.

Les sulfures d'or, d'argent et de cuivre, insolubles sans additifs, sont solubles sous la forme de complexes.

Pour le sulfure de cuivre SCu, dont la concentration maximale en solution aqueuse est de 0,33 mg/L, l'ajout d'ammoniaque comme additif permet d'atteindre une concentration aqueuse en Cu au moins égale à 0,2 g/L.

Pour le sulfure d'argent SAg₂, dont la concentration maximale en solution aqueuse est de soluble à 6,2.10⁻¹⁵ mg/L, l'ajout d'acide citrique ou d'un mélange d'acide citrique et d'ammoniaque comme additif permet d'atteindre une concentration aqueuse en Ag au moins égale à 0,2 g/L.

Pour le sulfure d'or S₃Au₂, la concentration aqueuse sans additif est indécelable. L'ajout soit de bichromate de potassium seul, soit d'un mélange équimolaire de bichromate de potassium et d'ammoniaque, permet d'atteindre une concentration aqueuse en Au au moins égale à 0,2 g/L.

Aussi décrite est l'utilisation comme substitut de parabène d'un métal de transition choisi parmi le cuivre, l'argent et l'or, étant sous forme de sulfure et sous forme de nanoparticules. lorsque le métal de transition est sous forme de nanoparticules de sulfure de cuivre, de nanoparticules de sulfure d'argent ou de nanoparticules de sulfure d'or, le métal de transition est, respectivement, au degré d'oxydation +II, +I et +III, c'est-à-dire Cu(ll), Ag(I) et Au(III).

Aussi décrite est l'utilisation, comme substitut de parabène, d'un métal de transition choisi parmi le cuivre, l'argent et l'or, étant au degré d'oxydation 0 et sous forme de nanoparticules.

Aussi décrite est l'utilisation comme substitut de parabène d'un métal de transition choisi parmi le cuivre, l'argent et l'or, étant sous forme de sel soluble.

Aussi décrite est l'utilisation d'un métal de transition dans une composition contenant de l'eau.

Avantageusement contenant de l'eau et un réducteur

Il est entendu par « *réducteur*» au sens de la présente invention, un composé capable de réduire le sel de métal de transition utilisé lors de la formation des nanoparticules du métal de transition tel que décrit ci-dessus et d'empêcher l'oxydation du métal de transition lors de son utilisation telle que décrite ci-dessus. Un exemple de réducteur au sens de l'invention est le glycérol en milieu basique (pH = 14), de préférence du glycérol en présence de NaOH. La réduction est effectuée à pH 14, et, une fois les nanoparticules de métaux formées, la solution est neutralisée et ramenée à pH 7. Selon un autre mode de réalisation avantageux, l'invention concerne l'utilisation d'un métal de transition dans une composition contenant de l'eau, ladite composition comprenant de l'ordre de 10⁻⁶ gramme de substitut de parabène par gramme de composition.
il est entendu par *« de l'ordre de 10⁻⁶ gramme* » une gamme de valeurs approximative, pouvant également autrement être formulée par une gamme s'échelonnant de 10⁻⁵ à 10⁻⁷ g.

Cette gamme de concentrations représente 100 à 10 000 fois moins de produit que lors de l'utilisation des parabènes. (les parabènes sont utilisés à des concentration de 4 à 8 pour 1000 hors d'Europe, et 3 fois moins en Europe)

Aussi décrite est l'utilisation d'un métal de transition dans une composition contenant de l'eau, dans laquelle la composition contenant de l'eau est une émulsion ou une suspension.

Aussi décrite est l'utilisation d'un métal de transition dans une composition contenant de l'eau, dans laquelle la composition contenant de l'eau est une émulsion huile-dans-l'eau ou eau-dans-l'huile.

Selon un mode de réalisation, le pourcentage d'eau par rapport à la quantité d'huile présent dans ces émulsions est de 65 à 99%, de préférence de 70 à 95%, en particulier de 70 à 80% d'eau.

Aussi décrite est l'utilisation comme substitut de parabène d'un métal de transition dans un produit alimentaire ou cosmétique ou pharmaceutique.

Aussi décrite est une composition sous la forme d'une émulsion huile-dans-l'eau ou eau-dans-l'huile, dépourvue de parabènes, comprenant du sulfure de cuivre, sulfure d'argent ou sulfure d'or ; en particulier, ladite composition comprenant du sulfure de cuivre.

Aussi décrite est une composition dans laquelle le sulfure de cuivre, sulfure d'argent ou sulfure d'or forme des nanoparticules.

Avantageusement la composition comprend le sulfure de cuivre, sulfure d'argent ou sulfure d'or, sous la forme de nanoparticules tels que définis ci-dessus, ladite composition comprenant également de l'eau et un additif.

La composition comprend le sulfure de cuivre, sulfure d'argent ou sulfure d'or, sous la forme d'un complexe tel que défini ci-dessus, ladite composition comprenant également de l'eau et un additif, ledit additif étant choisi parmi l'ammoniaque, l'acide citrique, le bichromate de potassium ou un mélange de ceux-ci.

La composition comprend le sulfure de cuivre, sulfure d'argent ou sulfure d'or, sous la forme de nanoparticules telles que définies ci-dessus, ladite composition comprenant également de l'eau et un additif, ledit additif étant du CTAB.

Aussi décrite est une composition sous la forme d'une émulsion huile-dans-l'eau ou eau-dans-l'huile, dépourvue de parabènes, comprenant du cuivre, de l'argent ou de l'or sous la forme de nanoparticules.
la composition sous la forme d'une émulsion huile-dans-l'eau ou eau-dans-l'huile, dépourvue de parabènes, comprenant du cuivre, de l'argent ou de l'or sous la forme de nanoparticules, comprend également un additif tel que du CTAB et un réducteur tel que du glycérol en milieu basique, en particulier du glycérol en présence de NaOH à pH 14. La réduction est effectuée à pH 14, et, une fois les nanoparticules de métaux formées, la solution est neutralisée et ramenée à pH 7.

Aussi décrit est un procédé de préparation des nanoparticules de métal de transition et nanoparticules de sulfure de métal de transition.

Le procédé de préparation des complexes solubles de sulfure de métal de transition de l'invention est décrit aux exemples 1 à 3.

Aussi décrit est un procédé (I) de préparation d'un bactéricide comprenant un métal de transition sous la forme de nanoparticules dudit métal de transition, ledit procédé comprenant une étape de réduction du métal de transition, en présence d'un additif, dans une solution aqueuse à pH 14 comprenant du glycérol. La réduction est effectuée à pH 14, et, une fois les nanoparticules de métaux formées, la solution est neutralisée et ramenée à pH 7

En particulier, le métal de transition est choisi dans la colonne IB de Mendeleïev, choisi parmi le cuivre (0), l'argent (0) et l'or (0).

En particulier, le métal de transition est sous la forme d'un sel métallique avant l'étape de réduction. Le sel métallique est en particulier choisi parmi du cuivre (II), notamment du nitrate de cuivre Cu(NO₃)₂, de l'argent (I), notamment du nitrate d'argent Ag(NO₃), et de l'or (III), notamment du chlorure d'or AuCl₃.

En particulier, la solution aqueuse à pH 14 comprend de l'eau, du glycérol et une base, notamment un hydroxyde, en particulier du NaOH. La réduction est effectuée à pH 14, et, une fois les nanoparticules de métaux formées, la solution est neutralisée et ramenée à pH 7
Dans le cadre de ce procédé (I), l'additif est un surfactant. Des exemples de surfactants incluent les RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, notamment les R1triméthylammoniumX. Le surfactant peut aussi être un un bromure d' ammonium triméthylR1 dans lequel R1 représente un alkyle de 6 à 20 atomes de carbone et notamment de 8, 9, 10, 11, 12, 13, 14, 15 ,16, 17, ou 18 atomes de carbone. Des exemples de surfactants incluent le CTAB (bromure de cétyltriméthylammonium ; Br[N(CH₃)₃(C₁₆H₃₃)]), le PEG-dithiol (Polyéthylène glycol comprenant des fonctions thiols terminales) et le PEG-600 (Polyéthylène glycol dont la masse molaire moyenne est de 600 g/mol).

La concentration en glycérol est comprise de 0,8 à 2,2 M, en particulier de 1 à 2 M, notamment de 1 à 1,85 M dans l'eau après l'étape de réduction du métal de transition.

Le procédé tel que décrit ci-dessus est effectué soit à 0 °C, soit à température ambiante, soit à une température de 60 à 100 °C, notamment de 80 à 100 °C.

Selon un mode de réalisation particulier, le procédé (I) comprend:
- une étape de formation d'une solution A comprenant le métal de transition sous la forme d'un sel métallique, un additif et de l'eau ;
- une étape de formation d'une solution aqueuse B à pH 14 comprenant du glycérol ;
- une étape de réduction du métal de transition par l'addition de la solution A à la solution aqueuse B pour former une suspension de nanoparticules ;
- une étape de neutralisation de la suspension de nanoparticules obtenue à l'étape précédente par l'addition d'un acide.

En particulier, dans l'étape de neutralisation du procédé (I) l'acide est un acide inorganique, notamment du HCl. Au sens de l'invention, il est entendu par « *neutralisation* » le passage d'une solution basique, dont le pH est compris de 8 à 14, à une solution à pH 7. La concentration en sel métallique lors de la formation de la solution A est comprise de 0,001 à 0,5 M, en particulier de 0,01 à 0,04 M, notamment 0,013 M, 0,023 M ou 0,04 M.

La concentration en additif lors de la formation de la solution A est comprise de 0,01 à 0,05 M, en particulier de 0,02 à 0,04 M, notamment 0,02 M ou 0,037 M.

Selon un mode de réalisation particulier, le procédé (I) comprend
- une étape de formation d'une solution A comprenant le métal de transition sous la forme d'un sel de cuivre (II), du CTAB et de l'eau ;
- une étape de formation d'une solution aqueuse B à pH 14 comprenant du glycérol ;
- une étape de réduction du métal de transition par l'addition de la solution A à la solution aqueuse B pour former une suspension de nanoparticules de cuivre (0) ;
- une étape de neutralisation de la suspension de nanoparticules de cuivre (0) obtenue à l'étape précédente par l'addition d'HCl.

Selon un mode de réalisation particulier, le procédé (I) comprend :
- une étape de formation d'une solution A comprenant le métal de transition sous la forme d'un sel d'argent (I), du CTAB et de l'eau ;
- une étape de formation d'une solution aqueuse B à pH 14 comprenant du glycérol ;
- une étape de réduction du métal de transition par l'addition de la solution A à la solution aqueuse B pour former une suspension de nanoparticules d'argent (0) ;
- une étape de neutralisation de la suspension de nanoparticules d'argent (0) obtenue à l'étape précédente par l'addition d'HCl.

Selon un mode de réalisation particulier, le procédé (I) comprend
- une étape de formation d'une solution A comprenant le métal de transition sous la forme d'un sel d'or (III), du CTAB et de l'eau ;
- une étape de formation d'une solution aqueuse B à pH 14 comprenant du glycérol ;
- une étape de réduction du métal de transition par l'addition de la solution A à la solution aqueuse B pour former une suspension de nanoparticules d'or (0) ;
- une étape de neutralisation de la suspension de nanoparticules d'or (0) obtenue à l'étape précédente par l'addition d'HCl.

Les conditions optimales pour la préparation de nanoparticules de métal de transition selon l'invention peuvent être déterminées par l'homme du métier, en particulier au vu des exemples 8 à 10 de la présente invention.

Aussi décrites sont des nanoparticules obtenues par le procédé (I).

Aussi décrit est un procédé (II) de préparation d'un bactéricide comprenant un métal de transition, ledit métal de transition étant sous la forme d'une nanoparticule de sulfure dudit métal de transition, ledit procédé comprenant une étape de précipitation d'un sel soluble du métal de transition en présence de sulfure de sodium Na₂S.

En particulier, dans le procédé (II) le sel soluble du métal de transition est choisi parmi du cuivre (II), notamment du sulfate de cuivre Cu(SO₄) ou du nitrate de cuivre Cu(NO₃)₂, de l'argent (I), notamment du nitrate d'argent Ag(NOs), et de l'or (III), notamment du chlorure d'or AuCl₃.

Dans le cadre de ce procédé (II), l'additif est un surfactant. Des exemples de surfactants incluent les RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium, en particulier le cétyltriméthylammonium ou l'octyltriméthylmammonium, notamment les R1triméthylammoniumX. Le surfactant peut aussi être un bromure d' ammonium triméthylR1 dans lequel R1 représente un alkyle de 6 à 20 atomes de carbone et notamment de 8, 9, 10, 11, 12, 13, 14, 15 ,16, 17, ou 18 atomes de carbone. Des exemples de surfactants incluent le CTAB (bromure de cétyltriméthylammonium ; Br[N(CH₃)₃(C₁₆H₃₃)]), le OTAB (le bromure d'octyltriméthylammonium ; Br[N(CH₃)₃(C₈H₁₇)]), le PEG-dithiol (Polyéthylène glycol comprenant des fonctions thiols terminales) et le PEG-600 (Polyéthylène glycol dont la masse molaire moyenne est de 600 g/mol).

Selon un mode de réalisation particulier, le procédé (II) comprend
- une étape de formation d'une solution A comprenant le métal de transition sous la forme d'un sel soluble du métal de transition, un additif et de l'eau ;
- une étape de formation d'une solution B comprenant du Na₂S et de l'eau ;
- une étape de précipitation du sel soluble du métal de transition par l'addition de la solution A à la solution B pour former une suspension de nanoparticules de sulfure métallique ;

En particulier, dans l'étape de neutralisation du procédé (II) l'acide est un acide inorganique, notamment du HCl. Au sens de l'invention, il est entendu par « *neutralisation* » le passage d'une solution basique, dont le pH est compris de 8 à 14, à une solution à pH 7.

La concentration en sel métallique lors de la formation de la solution A est comprise de 0,001 à 0,5 M, en particulier de 0,01 à 0,03 M, notamment 0,013 M, 0,023 M ou 0,025 M.

La concentration en additif lors de la formation de la solution A est comprise de 0,01 à 0,05 M, en particulier de 0,02 à 0,04 M, notamment 0,037 M. La concentration molaire de l'additif est 0,5 fois celle du sel métallique

La concentration en Na₂S est comprise de 0,001 à 0,02 M, en particulier de 0,003 à 0,02 M, notamment 0,003 M, 0,0065 M ou 0,0185 M dans l'eau après lors de l'étape de formation de la solution B. La concentration en Na₂S est la concentration stoechiométrique pour la formation du sel insoluble. Si le sel est SO₄Cu, Na₂S et SO₄Cu sont équimolaires. Selon un mode de réalisation particulier, le procédé (II) comprend
- une étape de formation d'une solution A comprenant le métal de transition sous la forme d'un sel soluble de cuivre (II), du CTAB et de l'eau ;
- une étape de formation d'une solution B comprenant du Na₂S et de l'eau ;
- une étape de précipitation du sel soluble de cuivre (II) par l'addition de la solution A à la solution B pour former une suspension de nanoparticules de sulfure de cuivre (II) ;

Selon un mode de réalisation particulier, le procédé (II) de l'invention comprend :
- une étape de formation d'une solution A comprenant le métal de transition sous la forme d'un sel soluble d'argent (I), du CTAB et de l'eau ;
- une étape de formation d'une solution B comprenant du Na₂S et de l'eau ;
- une étape de précipitation du sel soluble d'argent (I) par l'addition de la solution A à la solution B pour former une suspension de nanoparticules de sulfure d'argent (I) ;

La concentration en Na₂S est la concentration stoechiométrique pour la formation du sel insoluble. Si le sel est NO₃Ag, la concentration molaire de Na₂S est la moitié de celle de NO₃Ag

Selon un mode de réalisation particulier, le procédé (II) comprend
- une étape de formation d'une solution A comprenant le métal de transition sous la forme d'un sel soluble d'or (III), du CTAB et de l'eau ;
- une étape de formation d'une solution B comprenant du Na₂S et de l'eau ;
- une étape de précipitation du sel soluble d'or (III) par l'addition de la solution A à la solution B pour former une suspension de nanoparticules de sulfure d'or (III) ;

La concentration en Na₂S est la concentration stoechiométrique pour la formation du sel insoluble. Si le sel est Cl₃Au, la concentration molaire de Na₂S est 1,5 fois celle de Cl₃Au

Les conditions optimales pour la préparation de nanoparticules de sulfure métallique selon l'invention peuvent être déterminées par l'homme du métier, en particulier au vu des exemples de la présente invention.

Aussi décrites sont des nanoparticules obtenues par le procédé (II).

### FIGURES :

La Figure 1 représente une image HR-TEM des particules de SAg₂ selon l'Exemple 4.
La Figure 2 représente le spectre d'absorption UV-visible d'une suspension aqueuse de nanoparticules d'argent à une concentration de 0,2 wt% d'argent selon l'Exemple 8B.
La Figure 3 représente des images HR-TEM des particules d'argent de l'Exemple 8B. A, B, C et D correspondent à différentes échelles de taille.
La Figure 4 représente le spectre d'absorption UV-visible de la solution de nanoparticules d'argent à une concentration de 0,14 g.L⁻¹ (Exemple 8C) comparée à la solution de l'Exemple 8B.
La Figure 5 représente une image de microscopie HR-TEM réalisées sur la suspension à 0,14 g.L⁻¹ de l'Exemple 8C. A, B, C et D correspondent à différentes échelles de taille.
La Figure 6 montre des images HR-TEM de la solution à 2 g.L⁻¹ préparée dans l'exemple 8C. A et B correspondent à différentes échelles de taille.
La Figure 7 représente une image HR-TEM de la solution colloïdale de nanoparticules d'argent à 2 g.L⁻¹ obtenue après distillation sous vide d'une partie de l'eau d'une suspension à 0.2 g.L⁻¹, selon l'Exemple 8D.
La Figure 8 montre un histogramme de la distribution de taille des nanoparticules d'argent dans une suspension concentré à 2 g.L⁻¹ selon l'Exemple 8D. Le comptage de nanoparticules et la détermination de la taille de chacune d'elles sont réalisés sur un cliché de microscopie.
La Figure 9 montre une image HR-TEM de nanoparticules d'argent obtenues selon procédé 8.B mais en présence de bromure d'octyltriméthylammonium (OTAB) selon l'Exemple 8E.
La Figure 10 montre un histogramme de la distribution de taille des nanoparticules d'argent dans une suspension concentrée à 0.2 g.L⁻¹ préparée en présence de bromure d'octyltriméthylammonium (OTAB) selon l'Exemple 8E. Le comptage de nanoparticules et la détermination de la taille de chacune d'elles sont réalisés sur un cliché de microscopie.
La Figure 11 représente une image HR-TEM de la suspension de nanoparticules d'or selon l'Exemple 9.
La Figure 12 montre une image HR-TEM de la solution colloïdale comprenant les nanoparticules de cuivre selon l'Exemple 10.
La Figure 13 montre une image HR-TEM de la solution colloïdale comprenant les nanoparticules de cuivre selon l'Exemple 10.
La Figure 14 représente une image HR-TEM de la solution colloïdale comprenant les nanoparticules de soufre selon l'Exemple 11.

### EXEMPLES

### I ) Synthèse des complexes à titre de comparaison

### Exemple 1 (comparatif). Solution SCu

Dissoudre 126 mg de SO₄Cu dans 90 mL d'eau.

Dissoudre 140 mg de Na₂S dans 20 mL d'eau.

Mélanger les 90 mL de la solution de SO₄Cu avec 10 mL de la solution de Na₂S. On obtient un précipité de 75 mg de SCu. Le séparer par filtration.

Placer ce précipité dans 100 mL d'eau, dans un récipient avec agitateur.

En agitant constamment, y faire tomber goutte à goutte, très lentement une solution concentrée de NH₃ dans l'eau.

La concentration maximale de SCu dans l'eau est de 0,33 mg.L⁻¹. SCu devient plus soluble en solution aqueuse en présence d'ammoniaque. L'ajout d'ammoniaque au sulfure de cuivre permet d'atteindre une concentration aqueuse au moins égale à 0,2 g.L⁻¹ en Cu.

### Exemple 2 (comparatif). Solution SAg₂

Dissoudre 79 mg de NO₃Ag dans 97 mL d'eau.

Mélanger les 97 mL de la solution de NO₃Ag avec 3 mL de la solution de Na₂S. On obtient un précipité de 75 mg de SAg₂. Le séparer par filtration.

Placer ce précipité dans 100 mL d'eau, dans un récipient avec agitateur.

En agitant constamment, y faire tomber goutte à goutte, très lentement une solution concentrée de citrate d'ammonium (NH₄)₅(C₆H₄O₇)₂H₃.

La concentration maximale de SAg₂ dans l'eau est de 6,21.10⁻¹¹ g.L⁻¹. SAg₂ devient plus soluble en présence d'acide citrique, ou d'un mélange de citrate d'ammonium et d'acide citrique, ou d'un mélange de citrate d'ammonium et d'ammoniaque, le citrate d'ammonium étant formé par un mélange d'acide citrique et d'ammoniaque. L'ajout d'acide citrique, ou d'un mélange de citrate d'ammonium et d'acide citrique, ou d'un mélange de citrate d'ammonium et d'ammoniaque, au sulfure d'argent permet d'atteindre une concentration aqueuse au moins égale à 0,2 g.L⁻¹ en Ag.

### Exemple 3 (comparatif). Solution S₃Au₂

Dissoudre 77 mg de Cl₃Au dans 95 mL d'eau.

Mélanger les 95 mL de la solution de Cl₃Au avec 5 ml de la solution de Na₂S. On obtient un précipité de 62 mg de S₃Au₂. Le séparer par filtration.

Placer ce précipité dans 100 mL d'eau, dans un récipient avec agitateur.

En agitant constamment, y faire tomber goutte à goutte, très lentement une solution concentrée de bichromate de potassium. La concentration maximale d'Au₂S₃ dans l'eau est indécelable. La solubilité aqueuse de Au₂S₃ est augmentée en présence de bichromate de potassium, ou d'un mélange de bichromate de potassium et d'ammoniaque. L'ajout de bichromate de potassium, ou d'un mélange de bichromate de potassium et d'ammoniaque, au sulfure d'or permet d'atteindre une concentration aqueuse au moins égale à 0,2 g.L⁻¹ en Au.

### II) Synthèse des nanoparticules de sulfure métallique (Invention)

### Exemple 4. SAg₂ en nanoparticules

### Exemple 4A. SAg₂ en nanoparticules

Tous les réactifs sont utilisés tels que reçus, sans purification préalable.

L'eau utilisée pour préparer la solution est de l'eau ultra-pure dégazée et stockée sous Argon.

La réaction a lieu sous Argon, à température ambiante (20 °C).

La solution d'argent est manipulée dans le noir (protection sous aluminium).
- Pour la synthèse de 200 mL de solution colloïdale à 0,2 wt% (2 g.L⁻¹) de nanoparticules d'argent :
   Pour la préparation de la solution A, préparer 80 mL d'eau ultra-pure dans un ballon et dissoudre d'abord 1,35 g de bromure de cétyltriméthylammonium (CTAB ; 3,7.10⁻³ mol) dans l'eau puis 0,4 g d'Ag⁺ sous la forme d'AgNO₃ (2,3.10⁻³ mol) et ajuster à 100 mL avec de l'eau ultra-pure. Stocker à froid, dans le noir.

Pour la préparation de la solution B, préparer 50 mL d'eau ultra-pure dans un ballon, dissoudre 0,144 g de Na₂S (1,85.10⁻³mol) et ajuster à 100 mL avec de l'eau ultra-pure.

A température ambiante, dans le noir, sous une atmosphère d'argon et sous agitation (600 rpm), ajouter goutte à goutte la solution A à la solution B. Laisser réagir pendant 4h.

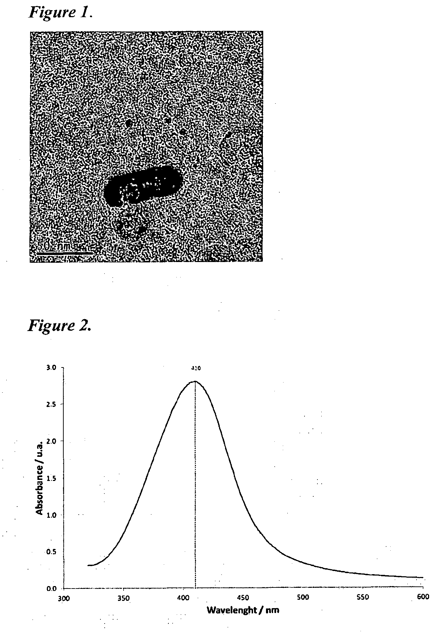

Les particules de SAg₂ sont caractérisées par HR-TEM, Figure 1. Elles présentent une large distribution de taille avec une présence majoritaire de particules de taille supérieure à 20 nm.

### Exemple 4B. SAg₂ en nanoparticules, effet de la température

Les nanoparticules de sulfure d'argent sont synthétisées selon l'exemple 4A, à 0 °C.

Il y a une différence peu significative sur l'activité bactéricide entre les nanoparticules obtenues à température ambiante et celles obtenues à 0°C (cf tableau 1 lignes R et O)

### Exemple 4C. SAg₂ en nanoparticules, effet du surfactant

Les nanoparticules de sulfure d'argent sont synthétisées selon l'exemple 4A, en utilisant du PEG-600 à la place du CTAB.

### Exemple 5. SCu en nanoparticules

### Exemple 5A. SCu en nanoparticules

Tous les réactifs sont utilisés tels que reçus, sans purification préalable.

L'eau utilisée pour préparer la solution est de l'eau ultra-pure dégazée et stockée sous Argon.

La réaction a lieu sous Argon, à température ambiante (20 °C).
- Pour la synthèse de 200 mL de solution colloïdale à 0,2 wt% (2 g.L⁻¹) de nanoparticules de cuivre :
   Pour la préparation de la solution A, préparer 80 mL d'eau ultra-pure dans un ballon et dissoudre d'abord 1,35 g de CTAB (3,7.10⁻³ mol) dans l'eau puis 0,4 g de Cu²⁺ sous la forme de CuSO₄ (2,5.10⁻³ mol) et ajuster à 100 mL avec de l'eau ultra-pure. Stocker à froid, dans le noir.

Pour la préparation de la solution B, préparer 50 mL d'eau ultra-pure dans un ballon, dissoudre 0,5 g de Na₂S (0,64.10⁻³ mol) et ajuster à 100 mL avec de l'eau ultra-pure.

A température ambiante, dans le noir, sous une atmosphère d'argon et sous agitation (600 rpm), ajouter goutte à goutte la solution A à la solution B. Laisser réagir pendant 4h.

### Exemple 5B. SCu en nanoparticules, effet de la température

Les nanoparticules de sulfure de cuivre sont synthétisées selon l'exemple 5A, à 0 °C.

Il n'y a pas de différence sur l'activité bactéricide entre les nanoparticules obtenues à température ambiante et celles obtenues à 0°C (cf lignes Q et T du tableau 1)

### Exemple 5C. SCu en nanoparticules, effet du surfactant

Les nanoparticules de sulfure de cuivre sont synthétisées selon l'exemple 5A, en utilisant du PEG-600 à la place du CTAB.

### Exemple 6. S₃Au₂ en nanoparticules

### Exemple 6A. S₃Au₂ en nanoparticules

Tous les réactifs sont utilisés tels que reçus, sans purification préalable.

L'eau utilisée pour préparer la solution est de l'eau ultra-pure dégazée et stockée sous Argon.

La réaction a lieu sous Argon, à température ambiante (20 °C).

La solution d'or est manipulée dans le noir (protection sous aluminium).
- Pour la synthèse de 200 mL de solution colloïdale à 0,2 wt% (2 g.L⁻¹) de nanoparticules d'or :
   Pour la préparation de la solution A, préparer 80 mL d'eau ultra-pure dans un ballon et dissoudre d'abord 1,35 g de CTAB (3,7.10⁻³ mol) dans l'eau puis 0,4 g de d'Au³⁺ sous la forme de AuCl₃ (1,3.10⁻³ mol) et ajuster à 100 mL avec de l'eau ultra-pure. Stocker à froid, dans le noir.

Pour la préparation de la solution B, préparer 50 mL d'eau ultra-pure dans un ballon, dissoudre 0,234 g de Na₂S (0,3.10⁻³ mol) et ajuster à 100 mL avec de l'eau ultra-pure.

A température ambiante, dans le noir, sous une atmosphère d'argon et sous agitation (600 rpm), ajouter goutte à goutte la solution A à la solution B. Laisser réagir pendant 4h.

### Exemple 6B. S₃Au₂ en nanoparticules, effet de la température

Les nanoparticules de sulfure d'or sont synthétisées selon l'exemple 6A, à 0 °C.

Les nanoparticules obtenues à température ambiante sont un peu plus efficaces que celles obtenues à 0°C (cf tableau 1 lignes P et S)

### III) Synthèse des nanoparticules de métaux de transition

### Exemple 7. Comment synthétiser des suspensions de nanoparticules métalliques dans l'eau ?

Les nanoparticules métalliques dans l'eau sont générées par la réaction d'un sel du métal et d'un réactif :
- le réactif étant un oxydant pour obtenir des nanoparticules d'oxydes métalliques ;
- le réactif étant un réducteur pour obtenir des nanoparticules métalliques.

De plus, les sels métalliques doivent être stabilisés dans des « cages » afin d'empêcher l'agglomération des nanoparticules en particules plus larges et plus stables.

Les cages sont généralement formées avec des surfactants possédant une partie hydrophile et une partie hydrophobe. Ces cages, en milieu aqueux, sont appelées micelles.

### Exemple 8. Nanoparticules d'argent, Ag

### Exemple 8.A Sélection des réactifs

Le sel d'argent disponible (et le plus commun) est AgNO₃. (Aldrich, 99%, M = 169.9 g.mol⁻¹, assay 63.4% Ag).

Le surfactant utilisé est le CTAB (bromure de cétyltriméthylammonium).

Le réducteur choisi est le glycérol.

L'utilisation du glycérol implique l'utilisation d'une base pour exalter ses propriétés de réducteur. Le NaOH a été utilisé à cet effet, pour produire une solution basique de glycérol dans l'eau. Ces solutions ont pour but de réduire le sel métallique d'Ag⁺ par la conversion du glycérol en dihydroxyacétone (DHA).

### Exemple 8.B Synthèse de 200 mL de solution colloïdale à 0,2 wt% (2 g.L⁻¹) de nanoparticules d'argent

Tous les réactifs sont utilisés tels que reçus, sans purification préalable. L'eau utilisée pour préparer la solution est de l'eau ultra-pure dégazée et stockée sous Argon. La réaction a lieu sous Argon, à température ambiante (20 °C). La solution d'argent est manipulée dans le noir (protection sous aluminium).

Pour la préparation de la solution A, préparer 80 mL d'eau ultra-pure dans un ballon et dissoudre d'abord 1,35 g de CTAB (3,7.10⁻³ mol) dans l'eau puis 0,4 g d'ion Ag⁺ sous la forme d'AgNOs (2,3.10⁻³ mol) et ajuster à 100 mL avec de l'eau ultra-pure. Stocker à froid, dans le noir.

Pour la préparation de la solution B, préparer 50 mL d'eau ultra-pure dans un ballon, dissoudre 34 g de glycérol (0,37 mol) puis 8 g de NaOH (0,2 mol) et ajuster à 100 mL avec de l'eau ultra-pure.

A température ambiante, dans le noir, sous une atmosphère d'argon et sous agitation (600 rpm), ajouter goutte à goutte la solution A à la solution B. Laisser réagir pendant 4h.

A la fin de la réaction, ajuster le pH à 7 par addition d'HCl commercial concentré (24 M). Le volume correspondant à ajouter est compris de 0,8 à 0,9 mL.

La solution résultante est analysée par spectroscopie UV-visible puis la morphologie et la taille des particules obtenues sont analysées par microscopie électronique à transmission haute résolution (HR-TEM).

La longueur d'onde d'absorption en UV-visible est spécifique de la taille et de la quantité des nanoparticules en suspension. L'analyse par spectroscopie UV-visible de la suspension colloïdale de nanoparticules d'argent à une concentration de 0,2 wt% est représentée sur la figure 2. La solution ne présente aucun dépôt, tout l'argent introduit initialement est sous forme colloïdale.

La distribution est mono-modale avec une longueur d'onde centrée à 410 nm, indiquant une taille de nanoparticules inférieure à 40 nm. La taille des nanoparticules est ensuite confirmée par microscopie HR-TEM.

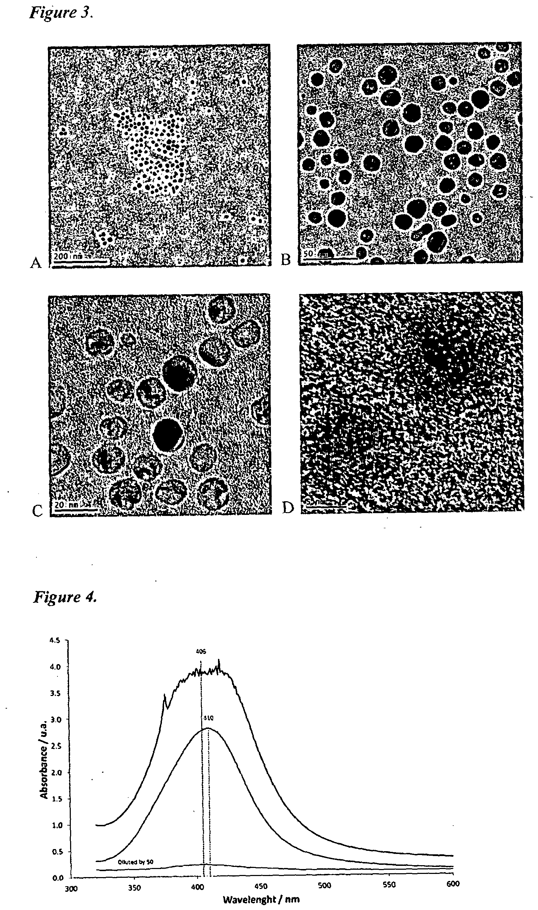

Les images obtenues par HR-TEM sont présentées sur la figure 3, à différentes échelles.

Les nanoparticules sont homogènes en taille, avec un diamètre moyen de 15 nm.

### Exemple 8.C Effet de la concentration en réactifs sur la formation des nanoparticules

Dans le but d'augmenter la concentration en nanoparticules et atteindre une concentration de 2 g.L⁻¹ (soit 0,2 wt%) en argent, la quantité de réactifs a été augmentée. Cependant toute la quantité de CTAB n'est pas soluble dans ces conditions et la majeure partie précipite. La stabilisation des nanoparticules d'argent n'est plus assurée.

Le spectre d'absorption UV-visible résultant d'une suspension qui a été diluée environ 50 fois afin d'éviter une saturation du spectre UV est présenté sur la figure 4. Il peut être déduit de la comparaison de ce spectre avec le spectre obtenu à l'exemple 8.B que la solution a une concentration en argent de 0,14 g.L⁻¹.

Une analyse par microscopie HR-TEM a été réalisée sur cette suspension diluée à 0,14 g.L⁻¹.La distribution est bimodale avec des nanoparticules possédant un diamètre d'environ 30 nm et des nanoparticules plus petites, d'un diamètre inférieur à 5 nm. Ce comportement est directement lié à un défaut de stabilisateur.

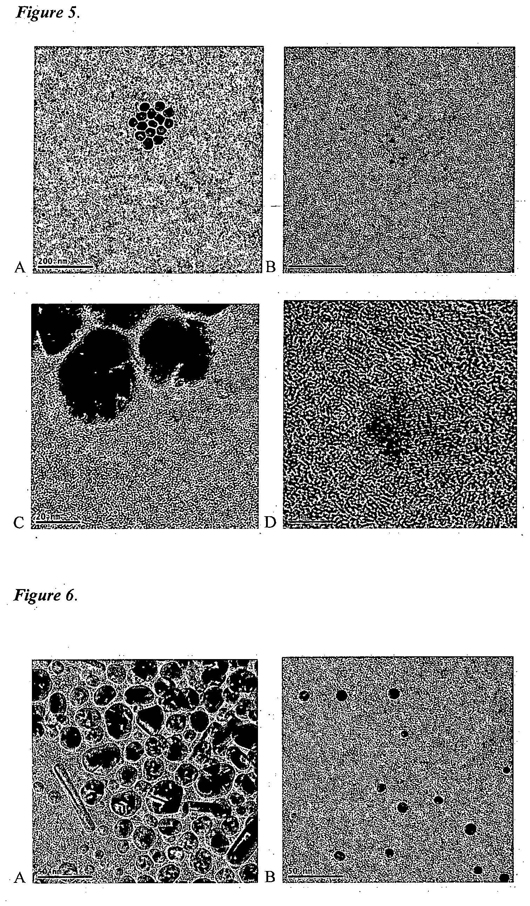

La solution obtenue selon le procédé 8.B mais avec une concentration de 2 g.L⁻¹ en argent, est de couleur dorée. Les images HR-TEM de cette solution 2 g.L⁻¹ (solution concentrée préparée en 8.C sont rapportées en Figure 6. Les particules obtenues ne sont pas homogènes. La figure 6A illustre la présence de nanoparticules avec un diamètre moyen de 30 nm, ces nanoparticules étant de formes diverses (bâtonnets, sphères). De petites nanoparticules sphériques, dont le diamètre moyen est compris de 5 à 10 nm, sont également présentent dans la solution, comme illustré sur la figure 6B.

### Exemple 8.D Effet de la concentration en argent sur la taille des nanoparticules d'argent.

La concentration en argent est obtenue par distillation sous vide de l'eau (p = 15 mbar à 20 °C) de la solution colloïdale initiale préparée selon l'exemple 8.B.

Une solution de nanoparticules d'argent de concentration 0,2 g.L⁻¹ et de taille inférieure à 15 nm (exemple 8.B) est concentrée par distillation sous vide de l'eau pour conduire à une concentration en argent de 2 g.L⁻¹.La solution résultante est trouble et dorée.

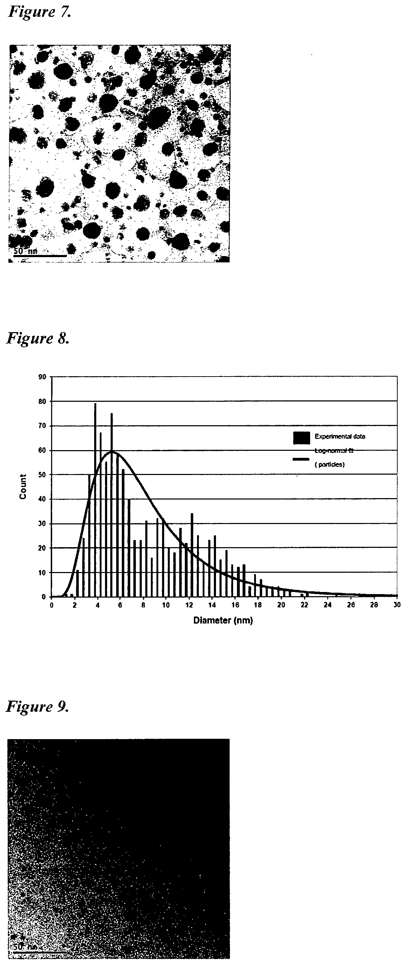

Les images obtenues par HR-TEM de cette nouvelle suspension sont présentées sur la figure 7.

Les nanoparticules généralement sphériques présentent une large distribution en taille (5 à 20 nm), Figures 7 et 8.

Il est cependant à noter que le CTAB précipite lors de l'élimination de l'eau.

### Exemple 8.E Effet de la nature du surfactant sur la formation des nanoparticules

Une solution de nanoparticule est réalisée selon l'exemple 8.B en utilisant du bromure d'octyltriméthylammonium (OTAB) à la place du bromure de cétyltriméthylammonium (CTAB).

La solution obtenue est verte. Un déplacement hypsochrome de la longueur d'onde du maximum d'absorption de cette solution est en effet observé lors de l'analyse par spectroscopie d'absorption UV-visible. Le Plasmon généré en surface est donc plus énergétique que lorsque les nanoparticules sont formées en présence de CTAB.

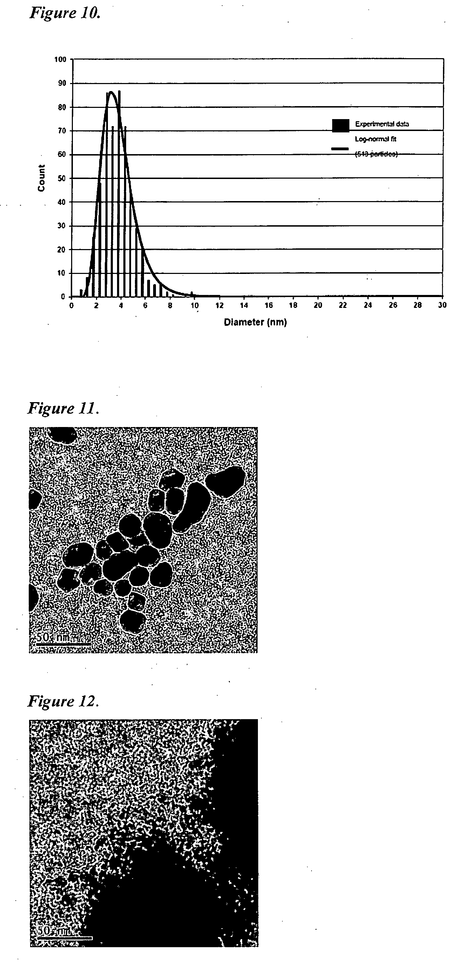

La figure 9 représente une image obtenue par microscopie HR-TEM de la solution verte. La taille des nanoparticules est très petite, d'un diamètre inférieur à 5 nm. Un diamètre moyen de 3,7 nm est déterminé par une analyse de la distribution en taille des particules de la solution (figure 10).

Le comptage de nanoparticules et la détermination de la taille de chacune d'elles sont réalisés sur un cliché de microscopie. 100 nanoparticules sont nécessaires afin que la distribution en taille déterminée soit représentative de l'ensemble de la population de la solution colloïdale de départ. Puis la distribution est ajustée par une loi log-normale.

### Exemple 8F. Effet de la nature du surfactant sur la formation des nanoparticules

Une solution de nanoparticule est réalisée selon l'exemple 8.B en utilisant du PEG-dithiol à la place du bromure de cétyltriméthylammonium (CTAB).

### Exemple 9. Nanoparticules d'or, Au

Tous les réactifs sont utilisés tels que reçus, sans purification préalable. L'eau utilisée pour préparer la solution est de l'eau ultra-pure dégazée et stockée sous Argon. La réaction a lieu sous Argon, à température ambiante (20 °C).

200 mL de solution colloïdale à 0,2 wt% (2 g.L⁻¹) en nanoparticules d'or sont synthétisés en suivant les étapes suivantes :
Pour la préparation de la solution A, préparer 80 mL d'eau ultra-pure dans un ballon et dissoudre d'abord 0,73 g de CTAB (2,0.10⁻³ mol) dans l'eau puis 0,4 g d'Au³⁺ sous la forme d'AuCl₃ (1,3.10⁻³ mol) et ajuster à 100 mL avec de l'eau ultra-pure. Stocker à froid.

Pour la préparation de la solution B, préparer 50 mL d'eau ultra-pure dans un ballon, dissoudre 0,2 mol de glycérol (18,4 g) puis 0,2 mol de NaOH (8 g) et ajuster à 100 mL avec de l'eau ultra-pure.

A température ambiante, sous une atmosphère d'argon et sous agitation (600 rpm), ajouter goutte à goutte la solution A à la solution B. Laisser réagir pendant 4h.

A la fin de la réaction, ajuster le pH à 7 par addition d'HCl commercial concentré (24 M). Le volume correspondant à ajouter est compris de 0,8 à 0,9 mL
La figure 11 représente une image HR-TEM des nanoparticules d'or obtenues. Leurs tailles varient entre 10 et 30 nm.

### Exemple 10. Nanoparticules de cuivre, Cu

### Exemple 10A. Synthèse des nanoparticules de cuivre, Cu

Les nanoparticules de cuivre sont synthétisées selon le protocole décrit à l'Exemple 8 B:
Tous les réactifs sont utilisés tels que reçus, sans purification préalable. L'eau utilisée pour préparer la solution est de l'eau ultra-pure dégazée et stockée sous Argon. La réaction a lieu sous Argon, à température ambiante (20 °C).

Pour la préparation de la solution A, préparer 80 mL d'eau ultra-pure dans un ballon et dissoudre d'abord 1,35 g de CTAB (3,7.10⁻³ mol) dans l'eau puis 0,4 g de Cu²⁺ sous la forme d'Cu(NO₃)₂ (Masse molaire : 187,56 g/mol : 0,75gCu(NO₃)₂ = 4.10⁻³ mol) et ajuster à 100 mL avec de l'eau ultra-pure. Stocker à froid, dans le noir.

Pour la préparation de la solution B, préparer 50 mL d'eau ultra-pure dans un ballon, dissoudre 0,37 mol de glycérol (34 g) puis 8 g de NaOH (0,2 mol) et ajuster à 100 mL avec de l'eau ultra-pure.

A température ambiante, dans le noir, sous une atmosphère d'argon et sous agitation (600 rpm), ajouter goutte à goutte la solution A à la solution B. Laisser réagir pendant 4h.

A la fin de la réaction, ajuster le pH à 7 par addition d'HCl commercial concentré (24 M). Le volume correspondant à ajouter est compris de 0,8 à 0,9 mL.

La figure 12 représente une image HR-TEM des nanoparticules de cuivre comprises dans la suspension colloïdale ainsi obtenue.

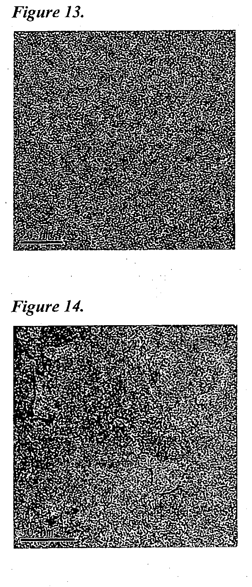

Des amas de petites particules sont observés démontrant que le surfactant ne permet pas une stabilisation complète des nanoparticules de cuivre. Quelques nanoparticules isolées sont également présentent comme illustré sur la figure 13. Ces nanoparticules sont homogènes et de petite taille, avec un diamètre d'environ 2 nm.

### Exemple 10B. Effet de la nature du surfactant sur la formation des nanoparticules de cuivre

Une solution de nanoparticule est réalisée selon l'exemple 10A en utilisant du PEG-600 à la place du bromure de cétyltriméthylammonium (CTAB).

### Exemple 11. Tentative de préparation de nanoparticules de soufre

Selon la procédure 8.B à partir de Na₂S et du PEG-600 comme surfactant : une solution colloïdale à 0,2 g.L⁻¹ en soufre a été obtenue.

Des objets homogènes en taille et de diamètre d'environ 5 nm mais difficiles à observer par microscopie HR-TEM en raison d'un faible contraste (figure 14), ont pu être observés. Ces objets du fait de leur taille ont été appelés nanoparticules de soufre.

### IV) Résultats bio

### Exemple 12. Test CMI sur les bactéries MRSA et MSSA : Matériels et Méthodes

Les bactéries MRSA (Meticilline Résistant Staphylococus Aureus ; ATCC 43300) et MSSA (Meticilline Sensible Staphylococus Aureus ; ATCC 29213) ont été incubées sur TSA 24 h à 37 °C. La standardisation des bactéries a été effectuée en réalisant un 0.5mc farland avec des colonies fraiches, représentant 1,5.10⁷ CFU.mL⁻¹ puis la suspension bactérienne a été diluée au 1/100^{ème} dans du milieu Mueller Hinton (MH) (sigma. 90922-500G) afin d'obtenir une concentration bactérienne finale (spf) correspondant à 1,5.10⁵ CFU.mL⁻¹.

Chaque essai sur particule a été réalisé sur une ligne d'une plaque 96 puits à fond rond (ref.353077) en duplicate. 50 µL de milieu MH ont été déposés dans les puits. 50 µL de la particule pure ont été déposés dans la première colonne (colonne 1) de la plaque puis des dilutions successives de raison 2 ont été réalisées en prenant 50 µl de chaque colonne redéposés dans la colonne suivante jusqu'à la dernière colonne (colonne 12). Les 50 µl de la dernière colonne ont été jeté. Puis 50 µL de la suspension bactérienne (spf) a été déposée dans chaque puit représentant une concentration finale bactérienne par puit de 0,75.10⁵ CFU.mL⁻¹. La concentration finale de la particule dans le premier puit (colonne 1) représentant une dilution finale au 1/4 par rapport à sa concentration initiale.

Deux contrôles négatifs ont été réalisés avec du MH pure, et avec 50 µL de MH et 50 µL de particule pure pour vérifier qu'il n'y avait pas de contamination. Un contrôle positif a été réalisé avec 50 µL de MH et 50 µL de la suspension bactérienne (spf).

La plaque a été incubée pendant 24 h à 37 °C dans une chambre humide.

Cette méthode étant la méthode de référence pour déterminer la CMI d'une particule, un contrôle de la CMI pour l'oxacilline a été réalisé sur ces mêmes souches MSSA et MRSA.

### Exemple 13. Test CMI sur les bactéries MRSA et MSSA : Résultats

La première cupule sans pousse de bactérie est déterminée comme étant la Concentration Minimale Inhibitrice (CMI) de la particule.

**Tableau 1. Test de Concentration Minimale Inhibitrice (CMI) des solutions aqueuses obtenues par l'utilisation de la présente invention sur les bactéries MRSA et MSSA. Les nanoparticules d'Ag(0) (selon l'exemple 8), de Cu(0) (selon l'exemple 10) et d'Au(0) (selon l'exemple 9) sont notées respectivement Ag, Cu et Au, et les nanoparticules de sulfure d'argent (selon l'exemple 4), de sulfure de cuivre (selon l'exemple 5) et de sulfure d'or (selon l'exemple 6) sont notées respectivement Ag₂S, CuS et Au₂S₃. CuSO₄ se rapporte à une solution de sel de cuivre soluble au sens de l'invention, c'est-à-dire à une solution de sulfate de cuivre dans l'eau. S représente une solution aqueuse de nanoparticules de soufre préparée selon l'exemple 11.**

| **Numéro** | **Particule/ quantité** | **CMI MSSA (g/L)** | **CMI MRSA (g/L)** |
|---|---|---|---|
| **Contrôle** | **Oxacilline** | 2,5.10⁻⁴ | 0,256 |
| **A** | **Ag** (selon Exemple 8B) | 7,81.10⁻⁴ | 7,81.10⁻⁴ |
| **B** | **Ag** (selon Exemple 8F) | 0,05 | 0,05 |
| **D** | **S** (selon Exemple 11) | 0,05 | 0,05 |
| **F** | **Cu** 0.2g/L (selon exemple 10A) | 4,88.10⁻⁵ | 9,71.10⁻⁵ |
| **H** | **Au** (selon Exemple 9) | 4,88.10⁻⁵ | 3,9.10⁻⁴ |
| **K** | **Cu** (selon Exemple 10B) | 0,025 | 0,05 |
| **L** | **Ag₂S** PEG-600 (selon Exemple 4C) | 0,0125 | |
| | | | Non déterminé |
| **N** | **CuS** PEG-600 (selon Exemple 5C) | 0,0125 | Non déterminé |
| **O** | **Ag₂S** (selon Exemple 4A) | 1,95.10⁻⁴ | 3,9.10⁻⁴ |
| **P** | **Au₂S₃** (selon Exemple 6A) | 2,44.10⁻⁵ | 4,88.10⁻⁵ |
| **Q** | **CuS** (selon Exemple 5A) | 9,71.10⁻⁵ | 9,71.10⁻⁵ |
| **R** | **Ag₂S** (selon Exemple 4B) | 1,95.10⁻⁴ | 1,95.10⁻⁴ |
| **S** | **Au₂S₃** (selon Exemple 6B) | 4,88.10⁻⁵ | 9,71.10⁻⁵ |
| **T** | **CuS** (selon Exemple 5B) | 9,71.10⁻⁵ | 9,71.10⁻⁵ |
| **V** | **CuSO₄** | 0,078 | 0,078-0,157 |
| **A+D** | 1/8 D + 7/8 A | 7,81.10⁻⁴ | 7,81.10⁻⁴ |
| **H+D** | 1/8 D + 7/8 H | 9,71.10⁻⁵ | 9,71.10⁻⁵ |
| **F+D** | 1/8 D + 7/8 F | 4,88.10⁻⁵ | 1,95.10⁻⁴ |
| **D+A+H** | 1/8 D + 3,5/8 A + 3,5/8 H | 9,71.10⁻⁵ | 1,95.10⁻⁴ |
| **D+A+F** | 1/8 D +3,5/8 A + 3,5/8 F | 9,71.10⁻⁵ | 9,71.10⁻⁵ |
| **D+H+F** | 1/8 D + 3,5/8 H + 3,5/8 F | 4,88.10⁻⁵ | 9,71.10⁻⁵ |
| **D+A+H+F** | 1/8 D+ 2/8 A + 2/8 H + 3/8 F | 4,88.10⁻⁵ | 9,71.10⁻⁵ |
| **D+K** | 1/8 D + 7/8 K | 0,025 | 0,05 |
| **O+P** | ½ O + ½ P | 9,71.10⁻⁵ | 9,71.10⁻⁵ |
| **P+Q** | ½ P + ½ Q | 4,88.10⁻⁵ | 9,71.10⁻⁵ |
| **O+Q** | ½ O + ½ Q | 7,81.10⁻⁴ | 1,95.10⁻⁴ |

Le contrôle représente bien la CMI attendue pour ces souches. Les CMI des différentes particules montrent un effet bactéricide pour la plupart sur les souches MSSA et MRSA à des concentrations différentes.

De plus, les nanoparticules de l'invention sont tous de meilleurs inhibiteurs de MRSA que l'oxacilline. L'oxacilline, utilisée ici comme référence, est un exemple de substance active utilisée en cas d'infection par des Staphylocoques.

Parmi les composés de l'invention, les nanoparticules de cuivre, les nanoparticules de sulfure d'or et les nanoparticules de sulfure de cuivre présentent les meilleurs taux d'inhibition, à la fois des bactéries MSSA et des bactéries MRSA.

### Exemple 14. Test CMI sur les bactéries Gram positif/négatif et sur les levures

La nanoparticule concernée est le sulfure de cuivre surfacté avec de CTAB, synthétisée selon l'exemple 5, la source de cuivre étant du nitrate de cuivre, Cu(NO₃)₂, et non du sulfate de cuivre. Les tests sont réalisés selon la méthode décrite à l'Exemple 12

Les activités sont mesurées par les CMI (Concentration Minimale Inhibitrice), l'index généralement utilisé, exprimé ici en mg par litre. Un mg par litre représente une ppm :

| | | |
|---|---|---|
| Bactéries Gram positif | *Staphylococcus Aureus* | 0,15 |
| | *Propionibacterium Acnes* | 0,07 |
| Bactérie Gram négatif | | |
| | *Pseudomonas Aeruginosa* | 1,2 |
| Levure | *Candida Albicans* | 0,32 |

Ces nanoparticules sont actives en particulier sur la bactérie qui produit l'acné : la concentration minimale inhibitrice contre le *propionibacterium acnés* est 0,07 mg par litre.

Les nanoparticules de sulfure de cuivre sont non seulement actives, mais sont de plus très bien tolérées, et cela d'autant plus que la concentration dans une crème anti-acné est faible : de l'ordre du ppm.

### Exemple 15

Il est à noter que dans tous les exemples ci-dessus faisant intervenir le CTAB, celui-ci peut être réduit à une concentration molaire égale à 0,5 fois celle du métal.

On a préparé également deux suspensions de nanoparticules de sulfure de cuivre avec un rapport CTAB/Cu respectivement de 1 et de 0.2 en suivant le protocole de l'exemple 5 et en utilisant les matières de départ ci-dessous

| | |
|---|---|
| CuSO₄.5H₂O | Masse mol : 249,69 g |
| Na₂S.9H₂O | Masse mol : 240,18 g |
| CTAB | Masse mol : 364,45 g |

Solution à 2 g.L⁻¹ de Cu (Masse mol : 63,55 g)
soit 0.2 g pour 100 mL ce qui correspond à 3.15 mmol de CuSO₄.5H₂O

Deux suspensions de CuS dans 100 mL d'eau ont été préparées à température ambiante.

### Suspension 1 rapport CTAB/Cu = 1 dans 100 mL

| | |
|---|---|
| 3.15 mmol de CuSO₄.5H₂O | Soit m = 0.786 g |
| 3.15 mmol de Na₂S.9H₂O | Soit m = 0.757 g |
| 3.15 mmol de CTAB | Soit m = 1.148 g |

Ces suspensions sont stables après 48 h à température ambiante

### Suspension 2 rapport CTAB/Cu = 0.5

| | |
|---|---|
| 3.15 mmol de CuSO₄.5H₂O | Soit m = 0.786 g |
| 3.15 mmol de Na₂S.9H₂O | Soit m = 0.757 g |
| 0.64 mmol de CTAB | Soit m = 0.230 g |

Ces suspensions sont stables après 48h à température ambiante

### Exemple 16

Les tests sur CuS à 1 de CTAB (suspension 2 de l'exemple 15) ont été faits par Groupe Ideatest.

La bactérie testée est le *propionibacterium Acnes,* responsable de l'acné.

Les résultats sont les suivants
La concentration létale est de 1 mg.L⁻¹ (une partie pour million)

Il existe deux autres mesures, déterminées également par Groupe Ideatest.

La mesure courante en Europe est la concentration 4 log (qui divise la population bactérienne par 10 000) en cinq minutes. Ceci est atteint pour le *propionibacterium acnés* avec 1 mg.L⁻¹.

La norme AFNOR EN 1276, à usage médical, bien plus rigide, est 5 log en cinq minutes (Elle divise la population bactérienne par 100 000) Ceci est atteint pour le *propionibacterium acnés* avec 2 mg.L⁻¹

Un contrôle de tolérance a été également fait par le Groupe Ideatest.. L'Europe interdisant maintenant les essais sur les animaux et sur les hommes en ce domaine, les laboratoires ont développé une méthode nouvelle. Elle consiste à cultiver des cellules de peau humaine, à les assembler en couches pour former une « peau reconstruite » et à tester sur cette peau reconstruite.

Ces essais ont été faits avec une concentration de 20 mg.L⁻¹ pour avoir une marge de sécurité très large. Contrairement à tous les anti-acnés actuellement sur le marché, la nanoparticule de l'invention ne produit aucune irritation.

D'autres tests ont été faits avec CuS à 10 de CTAB, (exemple 5) en concentration inhibitrice : ils ont donné les résultats suivants, en mg.L⁻¹. 1 mg.L⁻¹ correspond à une ppm, partie pour million :

| | | |
|---|---|---|
| Bactéries Gram positif | Staphylococus Aureus | 0,15 |
| | Propionibacterium Acnes | 0,07 |
| Bactérie Gram négatif | Escherichia Coli Pseudomonas Aeruginosa | 1,2 |
| Levures | Candida Albicans | 0,32 |

Ces résultats couvrent l'ensemble des familles de germes, gram positifs, gram négatifs et champignons notamment levures. Ils sont excellents.

### Exemple 17. Composition

Crème anti-acné, 80% d'eau 20% d'huile, contenant 2 ppm de SCu préparé avec du CTAB comme surfactant, le rapport de concentration molaire du CTAB/Cu était 0,2, préparation faite à température ambiante.

### Exemple 18 Tests de CuS à 1,15 de CTAB vis-à-vis du Staphylocoque Doré

Les mesures sur CuS à 1,15 de CTAB ont été effectuées par le CIRI :
Hospices Civils de Lyon - Centre International pour la Recherche en Infectiologie (CIRI) Hôpital de la Croix-Rousse
Centre de Biologie Nord - Laboratoire de Bactériologie - Bat O.
103 Grande Rue de la Croix Rousse, 69004 LYON, France
La bactérie testée est le Staphylococus Aureus.

Les résultats sont les suivants :
Le CIRI a recherché la CMI, Concentration Minimale Inhibitrice
D'une part sur le MRSA, Méticilline Résistant Staphylococcus Aureus
D'autre part sur le MSSA, Méticilline Sensible Staphylococcus Aureus

Dans les deux cas la CMI a été la même : 0,1 mg/L

### Exemple 19 Tests de CuS à 1,15 de CTAB vis à vis de la bactérie Propionibacterium acnés

Les tests sur CuS à 1,15 de CTAB ont été faits par le Groupe Ideatest (Idea Lab, Technopôle Montesquieu 5, rue Jacques Monod CS 60077 33652 MARTILLAC CEDEX).

La bactérie testée est le Propionibacterium Acnes, responsable de l'acné.

Les résultats sont les suivants :
Le produit a été testé aux concentrations : 0.07, 0.25, 0.5, 1 et 2 mg/l. Le suivi a été réalisé sur les temps 5 minutes, 24h et 6 jours.

Le microorganisme testé a été sorti de congélation (-80°C) et a été repiqué deux fois sur milieu approprié (TSA : Tryptic Soy Agar). Ce milieu a été incubé à 36°C (± 2.5°C) durant 6j en jarre anaérobie en présence d'anaerocult (Merck), P. acnes nécessitant des conditions anaérobies pour sa croissance.

La suspension à tester a été préparée extemporanément en eau physiologique stérile à partir de la culture sur milieu gélosé. La densité ainsi obtenue a été ajustée par mesure de la D.O. à 620 nm ; elle doit se situer aux environs de 10⁸ cellules/ml.

Le microorganisme à tester a été placé extemporanément dans un milieu liquide à double concentration favorable à sa croissance (TSB : Tryptic Soy Broth), puis immédiatement inoculé dans le produit à une densité finale comprise entre 10⁵ et 10⁶ Unité Formant Colonie/ml. Plus précisément, le contact entre la préparation microbienne et le produit à tester a été fait par addition volume à volume dans un flacon stérile en verre.

Après le prélèvement 5min, les incubations anaérobies des préparations de P. acnes ont été conduites en enceinte thermostatée durant 24h et 6j.

A l'issue des périodes d'incubation, des dénombrements ont été réalisés en milieu TSA, après dilution-neutralisation en LT100 sur des gammes de dilutions adaptées. Les incubations anaérobies des boîtes ensemencées ont été ensuite menées en enceintes thermostatées durant 6j.

L'activité bactéricide des produits est appréciée en comparant les densités obtenues au temps final (Tf) à celles notées au temps initial (T0). Le potentiel bactéricide s'estime à partir de l'abattement obtenu. Il doit être supérieur à 4 log pour les bactéries.

L'activité bactériostatique des produits est appréciée par l'éventuelle diminution de la croissance des germes microbiens entre témoin (Téf) et produit testé (Tf).

Le tableau suivant présente les densités obtenues aux :
- Temps initial (T0), les densités étant les mêmes pour le témoin et les tests et étant en l'occurrence de 5.0.10⁵ UFC/ml.
- Temps de suivi pour le témoin (Témoin) et les tests (0.07, 0.25, 0.5, 1 et 2mg/l).

Les résultats sont exprimés en UFC/ml.

| | **Témoin** | **0.07** | **0.25** | **0.5** | **1** | **2** |
|---|---|---|---|---|---|---|
| **T 5min** | 5.0.105 | 9.3.104 | 1.0.105 | 1.2.105 | 1.0.102 | <10 |
| **T 24h** | 7.0.105 | 3.9.105 | 3.7.105 | 7.4.104 | <10 | <10 |
| **T 6j** | 3.3.106 | 6.9.105 | 9.0.105 | 1.8.104 | <10 | <10 |

Sont ensuite calculés les abattements logarithmiques obtenus à chacun des temps de suivi en référence à la densité T0 :

| | **Témoin** | **0.07** | **0.25** | **0.5** | **1** | **2** |
|---|---|---|---|---|---|---|
| **T 5min** | 0.0 | 0.7 | 0.7 | 0.6 | 3.7 | 4.7 |
| **T 24h** | -0.1 | 0.1 | 0.1 | 0.8 | 4.7 | 4.7 |
| **T 6j** | -0.8 | -0.1 | -0.3 | 1.4 | 4.7 | 4.7 |

Sur la base de l'étude réalisée, le produit testé a présenté une activité bactéricide dès la concentration 1mg/l.

### Exemple 20 Tests de CuS à 1,15 de CTAB vis à vis de la bactérie Acinetobacter baumannii

Les tests sur CuS à 1,15 de CTAB ont été faits par l'Unité de Sécurité Microbiologique de l'Institut Pasteur de Lille (1 rue du Professeur Calmette BP 245 - 59019 LILLE Cedex).

La bactérie testée est Acinetobacter baumannii résistant aux Pénicillines, céphalosporines, carbapénèmes, quinolones, Aminoglycosides (gentamycine).

Les résultats sont les suivants :
Une série de dilutions successives au ½ du produit a été réalisée. Chaque dilution a été mise en contact avec la suspension bactérienne d'Acinetobacter baumannii (souche BAA 1792 résistante aux Pénicillines, céphalosporines, carbapénèmes, quinolones, Aminoglycosides (gentamycine)) et incubée à 37°C. Après 24 heures, les tubes présentant un trouble indiquent une croissance bactérienne ; la plus faible concentration de produit pour laquelle aucun trouble n'est observé est la concentration minimale inhibitrice (CMI).

Le contenu de chaque tube avec absence de trouble a alors été déposé sur gélose nutritive et incubé à 37°C. Après 24 heures, les géloses sur lesquelles aucune colonie n'est observée indiquent que le tube ne contenait plus de bactérie cultivable. La plus faible concentration de produit pour laquelle aucune colonie n'est observée est la concentration minimale bactéricide (CMB).

La CMI du produit vis-à-vis d'Acinetobacter baumannii a été estimée à 4,8 mg/L : pour des concentrations égales ou supérieures à cette valeur, la multiplication bactérienne est inhibée (effet bactériostatique).

La CMB a été estimée à 19,2 mg/L : pour des concentrations de produit supérieures ou égales à cette valeur, aucune population bactérienne résiduelle n'apparaît sur gélose (effet bactéricide).

La CMB a donc été retenue comme valeur à tester dans la norme NF EN 1276, ainsi qu'une concentration supérieure (30 mg/L) et une concentration inférieure estimée non bactéricide (0,3 mg/L).

### Exemple 21 Tests de CuS à 1,15 de CTAB vis à vis de la levure Candida albicans

Les tests sur CuS à 1,15 de CTAB ont été faits par l'Unité de Sécurité Microbiologique de l'institut Pasteur de Lille (1 rue du Professeur Calmette BP 245 - 59019 LILLE Cedex).

La levure testée est le *Candida albicans* résistante au Voriconazole, Itraconazole, Fluconazole, Anidulafungin
Les résultats sont les suivants :
Le mode opératoire utilisé pour la détermination des CMI et CML est identique à celui décrit dans l'exemple 19.

La CMI du produit vis-à-vis de Candida albicans a été estimée à 4,8 mg/L : pour des concentrations égales ou supérieures à cette valeur, la multiplication microbienne est inhibée (effet inhibiteur de croissance).

La CML a été estimée à 9,6 mg/L : pour des concentrations de produit supérieures ou égales à cette valeur, aucune population microbienne résiduelle n'apparaît sur gélose (effet microbicide).

La CML a donc été retenue comme valeur à tester dans la norme NF EN 1650, ainsi qu'une concentration supérieure (20 mg/L) et une concentration inférieure estimée non microbicide (0,3 mg/L).

### Exemple 22 Tests de CuS à 1,15 de CTAB vis à vis de la bactérie Escherichia coli

Les tests sur CuS à 1,15 de CTAB ont été faits par l'Unité de Sécurité Microbiologique de l'institut Pasteur de Lille (1 rue du Professeur Calmette BP 245 - 59019 LILLE Cedex).

La bactérie testée est *Escherichia coli productrice de bêta-lactamase à spectre étendu (BLSE), résistante aux carbapénèmes*

Les résultats sont les suivants :
Le mode opératoire utilisé pour la détermination des CMI et CML est identique à celui décrit dans l'exemple 19.

La CMI du produit vis-à-vis *d'Escherichia coli* a été estimée à 9,6 mg/L : pour des concentrations égales ou supérieures à cette valeur, la multiplication bactérienne est inhibée (effet bactériostatique).

La CMB a été estimée à 76,8 mg/L : pour des concentrations de produit supérieures ou égales à cette valeur, aucune population bactérienne résiduelle n'apparaît sur gélose (effet bactéricide).

### Exemple 23 Tests de l'innocuité de CuS à 1,15 de CTAB vis à vis de la peau

Les tests sur CuS à 1,15 de CTAB ont été faits par le Groupe Ideatest (Idea Lab, Technopôle Montesquieu 5, rue Jacques Monod CS 60077 33652 MARTILLAC CEDEX).

Le test a consisté en l'étude de l'irritation cutanée in vitro de l'échantillon sur épidermes humains reconstruits (modèle SkinEthic) selon la ligne directrice OCDE 439

L'échantillon a été testé à une concentration de 20 mg/mL.

Les résultats sont les suivants :
Les inserts (filtre + épiderme) ont été décollés doucement de la gélose et si nécessaire le dessous de l'inert a été essuyé sur du papier absorbant afin d'éviter de laisser de la gélose sur le filtre de polycarbonate. Les inserts ont été alors placés dans des puits (plaque de culture de 6 puits) préalablement remplis avec 1 mL de milieu de croissance à température ambiante (l'absence de bulles a été vérifiée). Les cultures ont été incubées à 37°C, 5% CO₂ une nuit.

16 µL ± 0.5 µL d'échantillon à tester ont été déposés à l'aide d'une micropipette à déplacement positif à la surface des tissus et un disque de nylon de 7.5 mm de diamètre a été appliqué délicatement sur la surface de l'épiderme à l'aide de pinces.

Les épidermes ont été incubés dans 0.3 mL de milieu de maintenance (plaque de 24 puits) à température ambiante pendant 42 minutes ± 1 minute.

Les disques de nylon ont été retirés et les épidermes ont été rincés avec 25 mL de PBS par épiderme (25 fois 1 mL à l'aide d'un distributeur).

Les épidermes ont été incubés dans 2 mL de milieu de croissance en plaque de 6 puits à 37°C, 5% CO₂ pendant 42 heures ± 1 heure (l'absence de bulles a été vérifiée).

A la fin de la période d'incubation, les plaques ont été agitées 2 minutes environ à 300 rotations par minute pour homogénéiser les médiateurs ou enzymes relargués dans le milieu de culture.

Le milieu de culture a été prélevé et congelé à -20°C ± 5°C pour le dosage éventuel de médiateurs ou enzymes.

Tous les épidermes ont été incubés dans 0.3 mL de milieu de maintenance à 1 mg/mL de MTT en plaque de 24 puits.

Après 3 heures ± 5 minutes d'incubation à 37°C, 5% CO₂, l'extérieur des inserts a été rincé avec 2 mL de PBS. L'extraction a été réalisée en plaçant les épidermes dans des puits remplis avec 0.8 mL d'isopropanol puis recouverts avec 0.7 mL d'isopropanol pendant 2 heures ± 5 minutes sous agitation douce à température ambiante. Les plaques ont été protégées par un film adhésif ou du parafilm pour éviter l'évaporation. Les épidermes ont été percés puis retirés des puits. La solution d'extraction a été homogénéisée par pipetages successifs puis l'absorbance a été mesurée en triplicat sur 200 µL d'extrait en plaques de 96 puits.

Les absorbances ont été mesurées à 540 nm contre un blanc constitué par l'isopropanol.

Le résultat est exprimé sous forme de pourcentage de viabilité comparé au contrôle négatif.

Le potentiel irritant de l'élément testé a été déterminé en accord avec le règlement CLP (règlement européen sur la classification, l'étiquetage et l'empaquetage des substances et des mélanges) et l'UN GHS (United Nations Globally Harmonized System of Classification and Labeling of Chemicals).

L'élément testé est considéré irritant pour la peau (Catégorie 2), si la viabilité relative moyenne, après 42 minutes d'exposition et 42 heures d'incubation, est inférieure ou égale à 50 % du contrôle négatif.

L'élément testé est considéré non irritant pour la peau (pas de catégorie), si la viabilité relative moyenne, après 42 minutes d'exposition et 42 heures d'incubation, est s à 50 % du contrôle négatif.

La viabilité moyenne observée pour l'échantillon testé est de 88.5%, celui-ci est donc non irritant.

### Exemple 24 Protocole de synthèse des nanoparticules ayant une structure constituée d'un coeur contenant un métal de transition, entouré d'une coquille de surfactant

Une solution A est formée de :
100 cc d'eau distillée
0,4 gr de Cu++, soit sous forme de SO₄Cu, soit (NO₃)₂Cu. Prendre garde au fait que ces deux produits sont livrés sous forme hydratée avec un nombre de molécules d'eau par Cu variable selon les fournisseurs. Il faut en tenir compte pour qu'il y ait 0,4 gr de Cu++
0,46 gr de CTAB
La solution B est formée de
100 cc d'eau distillée
0,49 gr de Na₂S. Prendre garde au fait que Na₂S peut être livré sous forme hydratée avec un nombre de molécules d'eau par Na₂S variable selon les fournisseurs. Il faut en tenir compte pour qu'il y ait 0,49 gr de Na₂S.

Verser goutte à goutte la solution B dans la solution A, en une heure, en agitant constamment la solution A.

L'ensemble de la synthèse est faite à l'ambiante.

### Exemple 25 Remplacement du CTAB par d'autres surfactants

### Principe :

On remplace le brome du CTAB par un autre acide (ex : nitrique, sulfurique, acétique, aminé, ...).

### Protocole de synthèse :

Dans une solution de CTAB verser la quantité stoechiométrique de poudre d'argent. Le bromure d'argent précipite. On l'élimine par filtration. Il reste une solution de CTA OH.

On verse dans cette solution la quantité stoechiométrique d'un acide (ex : nitrique, sulfurique, acétique, aminé, ...).

Le CTAX formé, où X est l'acide utilisé, est ensuite utilisé comme le CTAB pour produire des nanoparticules de métal de transition, notamment de SCu.

### Exemple 26 Comparaison de l'activité vis-à-vis du Staphylocoque Doré de différents métaux et nanoparticules selon l'invention

| | **CMI (Concentration Minimale Inhibitrice ) en PPB** | |
|---|---|---|
| **Echantillon testé** | **MRSA (Méticilline Résistant Staphylococcus Aureus)** | **MSSA (Méticilline Sensible Staphylococcus Aureus)** |
| Ag | 800 | 800 |
| SAg, t.a. | 200 | 50 |
| SAg, 0°C | 200 | 200 |
| Au | 400 | 50 |
| SAu, t.a. | 50 | 25 |
| SAu, 0°C | 100 | 50 |
| Cu | 100 | 50 |
| SCu, t.a. | 100 | 100 |
| SCu, 0°C | 100 | 100 |

| | | |
|---|---|---|
| t.a. : préparé à température ambiante 0°C : préparé à 0° degré | | |

### Exemple 27 Comparaison de l'activité vis-à-vis du Staphylocoque Doré de différents métaux et nanoparticules selon l'invention

**A:** Ag 0.2g/L
**B:** Ag 0.2g/L PEG dithiol pH=7
**C:** Ag2S/Ag 0.2g/L /CTAB
**D:** S 0.2g/L en présence de PEG-60 (S/PEG)
**E:** S 0.2g/L (S 0.2g/L /PEG-dithiol)
**F:** Cu 0.2g/L /CTAB 20% glycerol pH=7
**G:** Cu 0.2 g/L /CTAB pH=7
**H:** Au 0.2 g/L /CTAB 0.2g/L

| | **CMI (Concentration Minimale Inhibitrice ) en PPB** | |
|---|---|---|
| **Echantillon testé** | **MRSA (Méticilline Résistant Staphylococcus Aureus)** | **MSSA (Méticilline Sensible Staphylococcus Aureus)** |
| **A** | 7.81×10⁻⁴ | 7.81×10⁻⁴ |
| **B** | 0.05 | 0.05 |
| **C** | 1.95×10⁻⁴ | 4.88×10⁻⁵ |
| **D** | 0.05 | 0.05 |
| **E** | aucune inhibition | aucune inhibition |
| **F** | 9.71×10⁻⁵ | 4.88×10⁻⁵ |
| **G** | 9.71×10⁻⁵ | 4.88×10⁻⁵ |
| **H** | 3.9×10⁻⁴ | 4.88×10⁻⁵ |

## Revendications

1. Métal de transition sous forme insoluble, notamment sous forme d'un métal de transition au degré d'oxydation 0 ou sous forme de sulfure ou d'un oxyde du métal de transition,
ledit métal de transition étant choisi parmi l'or, l'argent et le cuivre,
ledit métal de transition sous forme insoluble étant mis en solution aqueuse par la formation de nanoparticules par ajout d'un surfactant,
ledit métal de transition étant contenu dans une structure constituée d'un coeur contenant ledit métal de transition, entouré d'une coquille de surfactant,
pour son utilisation dans le traitement de maladies bactériennes ou fongiques.

2. Métal de transition pour son utilisation selon la revendication 1, ladite utilisation étant une utilisation dans le traitement de l'acné.

3. Métal de transition pour son utilisation selon l'une des revendications 1 à 2, ledit surfactant étant choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium.

4. Métal de transition pour son utilisation selon l'une des revendications 1 à 3, le surfactant étant du bromure de cétyltriméthylammonium (CTBA).

5. Métal de transition pour son utilisation selon l'une des revendications 1 à 4, dans une composition contenant de l'eau,
ladite composition contenant de l'eau étant une solution aqueuse, ou une suspension, ou une émulsion.

6. Métal de transition sous forme insoluble, notamment sous forme d'un métal de transition au degré d'oxydation 0 ou sous forme de sulfure ou d'un oxyde du métal de transition,
ledit métal de transition étant choisi parmi l'or, l'argent et le cuivre,
ledit métal de transition sous forme insoluble étant mis en solution aqueuse par la formation de nanoparticules par ajout d'un surfactant,
ledit métal de transition étant contenu dans une structure constituée d'un coeur contenant ledit métal de transition, entouré d'une coquille de surfactant,
pour son utilisation comme antibactérien ou antifongique.

7. Métal de transition pour son utilisation selon la revendication 6, ladite utilisation étant une utilisation comme anti-acnéique.

8. Métal de transition pour son utilisation selon l'une des revendication 6 ou 7, ledit surfactant étant choisi parmi un RX, X étant un radical dérivé d'un acide minéral ou organique, en particulier NO₃⁻, SO₄⁻⁻, CH₃COO⁻, ou un halogène ou tout autre radical acide ; R étant un alkyle en C1 à C20 triméthylammonium.

9. Métal de transition selon l'une des revendications 6 à 8, dans une composition contenant de l'eau,
ladite composition contenant de l'eau étant une solution aqueuse, ou une suspension, ou une émulsion.

10. Composition pharmaceutique ou vétérinaire contenant un métal de transition selon l'une des revendications 1 à 9.

11. Composition pharmaceutique ou vétérinaire selon la revendication 10,
ladite composition comprenant comme excipients au moins une huile végétale,
la forme d'administration étant topique.

12. Composition pharmaceutique ou vétérinaire selon l'une des revendications 10 ou 11, ledit métal de transition étant à une dose de 1 mg à 1 g de métal de transition par litre de composition.

## Patentansprüche

1. Übergangsmetall in unlöslicher Form, insbesondere in Form eines Übergangsmetalls mit der Oxidationsstufe 0 oder in Form eines Sulfids oder eines Oxids des Übergangsmetalls,
wobei das Übergangsmetall aus Gold, Silber und Kupfer ausgewählt ist,
wobei das Übergangsmetall in unlöslicher Form in eine wässrige Lösung gebracht wird durch die Bildung von Nanopartikeln durch Zugabe eines Tensids,
wobei das Übergangsmetall in einer Struktur enthalten ist, die aus einem Kern besteht, der das Übergangsmetall enthält und von einer Schale aus Tensid umgeben ist,
zur Verwendung bei der Behandlung von Erkrankungen, die durch Bakterien oder Pilze verursacht werden.

2. Übergangsmetall zur Verwendung nach Anspruch 1, wobei diese Verwendung eine Verwendung bei der Behandlung von Akne ist.

3. Übergangsmetall zur Verwendung nach einem der Ansprüche 1 bis 2, wobei das Tensid ausgewählt ist aus einem RX, wobei X ein von einer Mineralsäure oder einer organischen Säure abgeleiteter Rest, insbesondere NO₃⁻, SO₄⁻, CH₃COO⁻, oder ein Halogen oder ein anderer Säurerest ist; R ein C1- bis C20-Alkyltrimethylammonium ist.

4. Übergangsmetall zur Verwendung nach einem der Ansprüche 1 bis 3, wobei das Tensid Cetyltrimethylammoniumbromid (CTBA) ist.

5. Übergangsmetall zur Verwendung nach einem der Ansprüche 1 bis 4 in einer wasserhaltigen Zusammensetzung,
wobei die wasserhaltige Zusammensetzung eine wässrige Lösung oder eine Suspension oder eine Emulsion ist.

6. Übergangsmetall in unlöslicher Form, insbesondere in Form eines Übergangsmetalls mit der Oxidationsstufe 0 oder in Form eines Sulfids oder eines Oxids des Übergangsmetalls,
wobei das Übergangsmetall aus Gold, Silber und Kupfer ausgewählt ist,
wobei das Übergangsmetall in unlöslicher Form in eine wässrige Lösung gebracht wird durch die Bildung von Nanopartikeln durch Zugabe eines Tensids,
wobei das Übergangsmetall in einer Struktur enthalten ist, die aus einem Kern besteht, der das Übergangsmetall enthält und von einer Schale aus Tensid umgeben ist,
zur Verwendung als antibakterielles oder antimykotisches Mittel.

7. Übergangsmetall zur Verwendung nach Anspruch 6, wobei die Verwendung eine Verwendung als Antiaknemittel ist.

8. Übergangsmetall zur Verwendung nach einem der Ansprüche 6 oder 7, wobei das Tensid ausgewählt ist aus einem RX, wobei X ein von einer Mineral- oder organischen Säure abgeleiteter Rest, insbesondere NO₃⁻, SO₄⁻, CH₃COO⁻, oder ein Halogen oder ein anderer Säurerest ist; R ein C1- bis C20-Alkyltrimethylammonium ist.

9. Übergangsmetall nach einem der Ansprüche 6 bis 8 in einer wasserhaltigen Zusammensetzung,
wobei die wasserhaltige Zusammensetzung eine wässrige Lösung oder eine Suspension oder eine Emulsion ist.

10. Pharmazeutische oder veterinärmedizinische Zusammensetzung, die ein Übergangsmetall nach einem der Ansprüche 1 bis 9 enthält.

11. Pharmazeutische oder veterinärmedizinische Zusammensetzung nach Anspruch 10, wobei die Zusammensetzung als Hilfsstoffe mindestens ein pflanzliches Öl enthält,
wobei die Verabreichungsform topisch ist.

12. Pharmazeutische oder veterinärmedizinische Zusammensetzung nach einem der Ansprüche 10 oder 11, wobei das Übergangsmetall in einer Dosis von 1 mg bis 1 g Übergangsmetall pro Liter der Zusammensetzung vorliegt.

## Claims

1. Transition metal in insoluble form, in particular in the form of a transition metal with oxidation state 0 or in the form of a sulfide or a transition metal oxide,
said transition metal being chosen from gold, silver and copper,
said transition metal in insoluble form being put into aqueous solution by the formation of nanoparticles by adding a surfactant,
said transition metal being contained in a structure consisting of a core containing said transition metal, surrounded by a surfactant shell,
for its use in the treatment of bacterial or fungal diseases, in particular acne.

2. Transition metal for its use according to claim 1, said use being a use in the treatment of acne.

3. Transition metal for its use according to any one of claims 1 to 2, said surfactant being in particular selected from an RX, X being a radical derived from a mineral or organic acid, in particular NO₃⁻, SO₄⁻, CH₃COO⁻, or a halogen or any other acidic radical, R being a C1-C20 alkyl trimethylammonium.

4. Transition metal for its use according to any one of claims 1 to 3, said surfactant being cetyltrimethylammonium (CTAB).

5. Transition metal for its use according to any one of claims 1 to 4, in a composition containing water, said composition containing water being an aqueous solution, or a suspension or an emulsion.

6. Transition metal in insoluble form, in particular in the form of a transition metal with oxidation state 0 or in the form of a sulfide or a transition metal oxide,
said transition metal being chosen from gold, silver and copper,
said transition metal in insoluble form being put into aqueous solution by the formation of nanoparticles by adding a surfactant,
said transition metal being contained in a structure consisting of a core containing said transition metal, surrounded by a surfactant shell,
for its use as an antibacterial or antifungal agent.

7. Transition metal for its use according to claim 6, said use being a use as an anti-acne agent.

8. Transition metal for its use according to any one of claims 6 to 7, said surfactant being in particular selected from an RX, X being a radical derived from a mineral or organic acid, in particular NO₃⁻, SO₄⁻, CH₃COO⁻, or a halogen or any other acidic radical, R being a C1-C20 alkyl trimethylammonium.

9. Transition metal for its use according to any one of claims 6 to 8, in a composition containing water, said composition containing water being an aqueous solution, or a suspension or an emulsion.

10. Pharmaceutical or veterinary composition containing a transition metal according to one of claims 1 to 9.

11. Pharmaceutical or veterinary composition according to claim 10, said composition comprising as excipients at least one vegetable oil, the form of administration of which being topical.

12. Pharmaceutical or veterinary composition according to one of claims 10 or 11, said transition metal being at a dose of 1 mg to 1 g of transition metal per liter of composition.
